# EUROPÄISCHE PATENTSCHRIFT

(11) **EP 2 744 907 B1**
(45) Veröffentlichungstag und Bekanntmachung des Hinweises auf die Patenterteilung: **22.03.2017**
(21) Anmeldenummer: 12738110.1
(22) Anmeldetag: 20.07.2012
(51) Int. Cl.: A61Q 5/00, A61Q 5/02, A61Q 5/12, A61Q 17/04, A61Q 19/00, A61K 8/44, A61Q 19/06, A61Q 19/08, A61K 8/99, A61K 36/04, C12P 13/00

(54) **VERFAHREN ZUR HERSTELLUNG VON 4-AMINOBUTTERSÄURE AUS ALGEN**
METHOD FOR PRODUCING 4-AMINOBUTYRIC ACID FROM ALGAE
PROCÉDÉ DE PRODUCTION D'ACIDE 4-AMINOBUTYRIQUE À PARTIR D'ALGUES

(30) Priorität: 18.08.2011 DE 102011110996
(43) Veröffentlichungstag der Anmeldung: 25.06.2014
(73) Patentinhaber: Evonik Degussa GmbH, 45128 Essen (DE)
(72) Erfinder: KÖHLER, Tim, 46284 Dorsten (DE); SCHILD, Jennifer, 42653 Solingen (DE); MENTEL, Matthias, 44357 Dortmund (DE); LERSCH, Peter, 46537 Dinslaken (DE); FARWICK, Mike, 45138 Essen (DE); WEITEMEYER, Christian, 37136 Seeburg (DE)
(86) Internationale Anmeldenummer: PCT/EP2012/064261
(87) Internationale Veröffentlichungsnummer: WO 2013/023873

(56) Entgegenhaltungen:
- WO-A1-2007/007989
- WO-A2-2008/081095
- US-A1- 2006 198 809
- BEALE S I CORNEJO J: "Biosynthesis of phycobilins. Ferredoxin-mediated reduction of biliverdin catalyzed by extracts of cyanidium caldarium", JOURNAL OF BIOLOGICAL CHEMISTRY, AMERICAN SOCIETY FOR BIOCHEMISTRY AND MOLECULAR BIOLOGY, US, Bd. 266, Nr. 33, 25. November 1991 (1991-11-25), Seiten 22328-22332, XP002958825, ISSN: 0021-9258
- ITO ET AL: "GABA-synthesizing enzyme, GAD67, from dermal fibroblasts: Evidence for a new skin function", BIOCHIMICA ET BIOPHYSICA ACTA - GENERAL SUBJECTS, ELSEVIER SCIENCE PUBLISHERS, NL, Bd. 1770, Nr. 2, 29. Dezember 2006 (2006-12-29), Seiten 291-296, XP005818422, ISSN: 0304-4165, DOI: 10.1016/J.BBAGEN.2006.09.017
- DATABASE WPI Week 199030 Thomson Scientific, London, GB; AN 1990-227348 XP002683807, & JP 2 154681 A (CHLORELLA KOGYO KK) 14. Juni 1990 (1990-06-14)

## Beschreibung

### Gebiet der Erfindung

Gegenstand der Erfindung ist ein Verfahren zur Herstellung von 4-Aminobuttersäure, auch gamma-Aminobuttersäure, kurz GABA, genannt, ein Verfahren zur Herstellung eines Extraktes enthaltend 4-Aminobuttersäure, ein Verfahren zur Herstellung einer kosmetischen Formulierung enthaltend 4-Aminobuttersäure, sowie die Verwendung von *Cyanidium caldarium-Zellen* oder Zellbestanteilen in kosmetischen Anwendungen.

### Stand der Technik

Die positiven Auswirkungen von GABA auf Haut wurde erstmal von Ito et al. in Biochim Biophys Acta. 2007 Feb; 1770(2):291-6 beschrieben, als die Expression des sonst nur als neuronal lokalisiert beschriebenen GAD67 in menschlichen dermalen Fibroblasten, welches die GABA-Synthese katalysiert, aufgezeigt wurde.
Es wurde nachgewiesen, dass GABA in der Lage ist, die Hyaluronsäure-Synthese in Fibroblasten heraufzuregulieren, und dass GAD67 einen positiven Einfluss auf die Kollagen-Synthese ausübt.
Kosmetika enthaltend GABA (INCI: Aminobutyric Acid) finden sich in den Bereichen der Farbkosmetik, Deodorantien, Feuchttücher, Hautpflege und Duschprodukte. Hier wird GABA als hautidentischer Stoff mit Muskel-entspannender Wirkung, Hautaufhellender Wirkung, feuchtigkeitsspendender Wirkung und als Stoff mit stimulierender Wirkung auf den Aufbau von Kollagen und Matrikin beschrieben.
Einsatzgebiete sind Anwendungen wie Anti-Aging, Anti-Wrinkle, Straffung und Glättung von Lippenlinien, Reduzierung von Mimikfalten, Reduzierung von dünnen Falten und Krähenfüßen. Das verwendete GABA entstammt der mikrobiellen pflanzlichen Fermentation. Hierbei werden zum Beispiel Reiskeime bei 37 °C über mehrere Jahre fermentiert.

Die Synthese von GABA ist bisher fast ausnahmslos nur in höheren Organismen vorkommend beschrieben worden.
In Algen wurde bisher nur das Vorkommen von GABA-Mimetika in beispielsweise *Porphyra* von Morse und Morse in Hydrobiologia, 116-117 (1): 155-158 beschrieben.

Aufgabe der Erfindung war es, eine kosmetische Formulierung bereitzustellen, welche GABA enthält, wobei die Quelle des GABA auf einfach zu kultivierenden und schnell wachsenden Mikroorganismen basiert.

### Beschreibung der Erfindung

Überraschenderweise wurde gefunden, dass die Alge *Cyanidium caldarium* hohe Mengen an GABA enthält und sich diese hervorragend zur Herstellung von GABA sowie eines Kosmetikums verwenden lässt.
Dies ist insbesondere deswegen überraschend, da es bisher für unmöglich gehalten wurde, eine Alge zu finden, die GABA zu synthetisieren vermag.

Gegenstand der vorliegenden Erfindung sind daher ein Verfahren zur Herstellung von GABA sowie eines GABA-haltigen Kosmetikums unter Einsatz von *Cyanidium caldarium*-Zellen.
Ein weiterer Gegenstand der Erfindung ist die Verwendung von *Cyanidium caldarium*-Zellen oder Zellbestanteilen in einer kosmetischen Formulierung

Vorteil der vorliegenden Erfindung ist die Nachhaltigkeit aufgrund des Einsatzes eines nachwachsenden Rohstoffes.
Ein weiterer Vorteil ist die einfache Bereitstellung des GABAs aufgrund der einfachen Aufarbeitbarkeit der *Cyanidium caldarium-*Zellen*.*
Noch ein Vorteil der vorliegenden Erfindung ist es, dass *Cyanidium caldarium-*Zellen keine für ein Kosmetikum störenden Nebenkomponenten enthalten.
Noch ein Vorteil der vorliegenden Erfindung ist es, dass *Cyanidium caldarium-*Zellen weitere für ein Kosmetikum vorteilhafte Nebenkomponenten enthalten, wie beispielsweise Nährstoffe, Mineralstoffe, nicht-essentielle und essentielle Aminosäuren, Ornithin und Polyphenole.
Noch ein Vorteil der vorliegenden Erfindung ist es, dass das erfindungsgemäße Kosmetikum Markergene der extrazellulärer Matrix hochzuregulieren vermag. Insbesondere Fibrillin und Elastin, die wichtigsten Strukturkomponenten von elastischen Fasern, werde vorteilhaft gleichmäßig hochreguliert.
Noch ein Vorteil ist es, dass das erfindungsgemäße Kosmetikum eine antioxidative Wirkung und einen ernährenden Effekt auf Haut aufweist.

Noch ein Vorteil ist, dass das erfindungsgemäße Kosmetikum sich als wirksam für unterschiedlichste Hauttypen, vor allem für gealterte Haut, erweist und sich entzündungshemmend und hautberuhigend auswirkt.

Gegenstand der vorliegenden Erfindung ist somit ein Verfahren zur Herstellung von 4-Aminobuttersäure umfassend die Verfahrensschritte
A) Kultivieren von *Cyanidium caldarium*-Zellen in einem wässrigen Medium,
B) Aufschließen der *Cyanidium caldarium-*Zellen, gegebenenfalls
C) Abtrennen der festen Zellbestandteile unter Erhalt eines Extraktes und gegebenenfalls
D) weiteres Aufreinigen der 4-Aminobuttersäure.

Unter dem Begriff "*Cyanidium caldarium*" ist im Zusammenhang mit der vorliegenden Erfindung ist die als *Cyanidium caldarium* (Tilden) Geitler klassifizierte Alge aus der Familie der *Cyanidiaceae* zu verstehen.
Unter dem Begriff "wässrig" ist ein Wassergehalt von mindestens 50 Gew.-% zu verstehen, bezogen auf die Gesamtzusammensetzung, worauf sich "wässrig" bezieht, hier speziell das Medium.
Unter dem Begriff "Aufschließen der Zellen" ist ein Zerstören der Zellmembran, insbesondere der äußeren Zellmembran, zu verstehen.
Unter dem Begriff "Extrakt" sind flüssige Mischungszusammensetzungen verschiedener Komponenten zu verstehen, wobei sich diese
Mischungszusammensetzungen aus den *Cyanidium caldarium*-Zellen isolieren lassen. Unter dem Begriff "Zellbestandteile" sind Mischungszusammensetzungen verschiedener Komponenten zu verstehen, welche sich aus *Cyanidium caldarium-*Zellen isolieren lassen.
Alle angegebenen Prozent (%) sind, wenn nicht anders angegeben, Massenprozent.

Erfindungsgemäß bevorzugte *Cyanidium caldarium-*Zellen sind Zellen des Stammes SAG 16.91.

Es ist erfindungsgemäß bevorzugt, dass die *Cyanidium caldarium-*Zellen in Verfahrensschritt A) bei einer erhöhten Temperatur, daher in ein einem bevorzugten Temperaturbereich von 20 °C bis 60 °C, insbesondere 35 °C bis 45 °C kultiviert werden. Bevorzugt ist es, dass die Kultivierung bei erhöhter Temperatur über einen Zeitraum von 7 Tage bis 192 Tage, insbesondere 28 Tage bis 56 Tage, erfolgt. Hierdurch wird es möglich, einen Extrakt oder ein Kosmetikum bereitzustellen, welches neben GABA Polyamine wie z. B. Spermin, Spermidin und Norspermin enthält.
Das wässrige Medium in Verfahrensschritt A) weist bevorzugt einen pH-Wert bei 25 °C von 1,5 bis 6, bevorzugt von 2 bis 3, auf.

Das Aufschließen der Zellen in Verfahrensschritt B) kann durch Behandlung einer Suspension der Algenkultur mit Ultraschall, durch Anwendung eines osmotischen Schocks, durch Hochdruckhomogenisation (z. B. French-Press, Manton-Gaulin-Homogenisator), durch Heisswasserextraktion, durch Einsatz hoher Scherkräfte (z. B. Ultra-Turrax, Potter-Elvehjem Verfahren), durch Nassvermahlen in Rührwerkkugelmühlen, durch Druckentspannen, mit Hilfe von Enzymen, wie beispielsweise Polysaccharidasen wie Glucanase, Hemicellulase, Cellulase oder auch kommerziellen Enzymen wie SP-311 (novo) oder durch beliebige Kombinationen dieser vorgenannten Verfahren erfolgen.

Die Zellen werden in Verfahrensschritt B) bevorzugt in einem wässrigen Medium aufgeschlossen, so dass bevorzugt ein wässriger Extrakt erhalten wird. Besonders bevorzugt entspricht das wässrige Medium des Verfahrensschritts B) dem Medium des Verfahrensschritts A), insbesondere handelt es sich um dasselbe Medium.

Insbesondere ein Heisswasseraufschluss kombiniert mit einem enzymatischen Zellaufschluss ist in Verfahrensschritt B) bevorzugt.

Das Abtrennen der festen Zellbestandteile in Verfahrensschritt C) kann beispielsweise durch Zentrifugation, Sedimentation oder Filtration erfolgen.

Die weitere Aufreinigung des GABAs kann erfolgen wie beispielsweise in Carmona et al., Acta Pharmacol Toxicol (Copenh). 1980 Mar;46(3):235-40 beschrieben erfolgen.

Ein weiterer Gegenstand der vorliegenden Erfindung ist auch ein Verfahren zur Herstellung eines Extraktes enthaltend 4-Aminobuttersäure umfassend die Verfahrensschritte
A) Kultivieren von *Cyanidium caldarium-*Zellen in einem wässrigen Medium,
B) Aufschließen der *Cyanidium caldarium*-Zellen, gegebenenfalls
C) Abtrennen der festen Zellbestandteile unter Erhalt eines Extraktes,
wobei die Verfahrensschritte A) bis C) dem erstgenannten Verfahren der vorliegenden Erfindung entsprechen und entsprechende bevorzugte Ausführungsformen ebenso bevorzugt sind.
Bevorzugt besteht das erfindungsgemäße Verfahren zur Herstellung eines Extraktes aus den Schritten A), B) und C).

Ein weiterer Gegenstand der vorliegenden Erfindung ist auch ein Verfahren zur Herstellung einer kosmetischen Formulierung enthaltend 4-Aminobuttersäure umfassend die Verfahrensschritte
A) Kultivieren von *Cyanidium caldarium*-Zellen in einem wässrigen Medium,
B) Aufschließen der *Cyanidium caldarium-*Zellen, gegebenenfalls
C) Abtrennen der festen Zellbestandteile unter Erhalt eines Extraktes und
E) Einarbeiten der aufgeschlossenen *Cyanidium caldarium-*Zellen aus Verfahrensschritt B) und/oder des Extraktes aus Verfahrensschritt C) in eine kosmetische Formulierung,
wobei die Verfahrensschritte A) bis C) dem erstgenannten Verfahren der vorliegenden Erfindung entsprechen und entsprechende bevorzugte Ausführungsformen ebenso bevorzugt sind.
Bevorzugt besteht das erfindungsgemäße Verfahren zur Herstellung einer kosmetischen Formulierung aus den Verfahrensschritten A), B), C) und E). Bevorzugt wird in Verfahrensschritt E) nur der Extrakt aus Verfahrensschritt C) eingearbeitet.

Ein weiterer Gegenstand der vorliegenden Erfindung ist die Verwendung von *Cyanidium caldarium*-Zellen oder Zellbestandteilen in einer kosmetischen Formulierung.

Die kosmetische Formulierung erweist sich als wirksam für unterschiedlichste Hauttypen, vor allem für gealterte Haut (chronologisch gealterte Haut, photo-gealterte Haut, sackende Haut, schlaffe Haut).
Die Verwendung der kosmetischen Formulierung führt generell zu einer Verbesserung der Hautstruktur, wodurch die erfindungsgemäße kosmetische Formulierung als universeller Anti-Aging Wirkstoff verwendbar wird. Insbesondere ist Die kosmetische Formulierung ist verwendbar für formgebende kosmetische Behandlungen, wie z. B. an Kinn, Brust, Gesäß und Bauch.

Weitere positive Effekte der kosmetischen Formulierung beinhalten u.a. eine Verminderung der Haut-Rauhigkeit, der Haut-Schuppigkeit, der Faltentiefe, sowie eine Verstärkung der Hautelastizität, der Haut-Straffheit und der Haut-Dicke.

Die topische Applikation der kosmetischen Formulierung auf der Haut führt zu einer Verminderung der Anzeichen von gestresster, irritierter, geröteter und entzündeter Haut. Dadurch ist die erfindungsgemäße kosmetische Formulierung in besonderem Maße für kosmetische Produkte zur Beruhigung gereizter Haut, sensibler Haut geeignet. Die entzündungshemmende und hautberuhigende Wirkung von der kosmetischen Formulierung favorisiert auch eine Anwendung für hautberuhigende After-Shave Lotionen.

Die kosmetische Formulierung hat eine protektive Wirkung vor schädlichen intrinsischen sowie extrinsischen Einflußfaktoren (Umweltnoxen), wodurch einer Schädigung zellulärer Makromoleküle sowie der epidermalen Lipidbarriere vorgebeugt werden kann.

Eine hautberuhigene Wirkung von der kosmetischen Formulierung ist auch auf geröteter Haut zu beobachten, die infolge von Sonneneinstrahlung Sonnenbrand bzw. Hautrötungen (Erytheme) aufweist. Insofern ist die kosmetische Formulierung auch als Wirkstoff für Sonnenschutz und After-Sun-Produkte einsetzbar.

Daneben eignet sich die kosmetische Formulierung zur Pflege und zum Schutz von Haut, insbesondere solcher Haut, die in der Folge bestimmter Hauterkrankungen eine Schwächung der epidermalen Barrierefunktion aufweist, und damit verbunden einen erhöhten transepidermalen Wasserverlust bzw. eine verminderte Hautfeuchte aufweist. Entsprechende Hauterkrankungen beinhalten u.a. Xerosis, atopische Dermatitis, Kontakt-Dermatitis, Psoriasis, Ichthyosis, Acanthosis, Schuppen, Photodermatitis, Erythema, und Keratinisierungsstörungen bzw. Verhornungsdefekte. Ebenfalls geeignet ist die kosmetische Formulierung zur Anwendung auf Haut, um bestimmte Auswirkungen wie trockener, juckender und schuppiger Haut abzumildern, die beispielsweise als Folge von Autoimmunerkrankungen (z.B. Psoriasis) auftreten.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Folgende Abbildungen sind Bestandteil der Beispiele:
Abbildung 1: Expression des COL1A1 Gens in humanen dermalen Fibroblasten 24 h nach Applikation von 100 ppm (= 100 µg/ml) *Cyanidium caldarium* Extrakt (Konzentration bezogen auf Trockenmasse des Extrakts) bzw. reinem Medium (Vehikel) relativ zur Genexpression von GAPDH.
Abbildung 2: Expression verschiedener Gene in humanen dermalen Fibroblasten 24 h nach Applikation von reinem Medium (Vehikel), TGF-ß (10 ng/ml, Positivkontrolle) bzw. 100 ppm (= 100 µg/ml) *Cyanidium caldarium* Extrakt (Konzentration bezogen auf Trockenmasse des Extrakts) relativ zur Genexpression von GAPDH.
Abbildung 3: Elastin Proteingehalt in humanen dermalen Fibroblasten 24 h nach Applikation von reinem Medium (Vehikel), 100 ppm (= 100 µg/ml) *Cyanidium caldarium* Extrakt (Konzentration bezogen auf Trockenmasse des Extrakts) bzw. TGF-ß (10 ng/ml, Positivkontrolle) relativ zum Gesamtproteingehalt der Zellen.
Abbildung 4: Lumican Proteingehalt in humanen dermalen Fibroblasten 24 h nach Applikation von reinem Medium (Vehikel), 100 ppm (= 100 µg/ml) *Cyanidium caldarium* Extrakt (Konzentration bezogen auf Trockenmasse des Extrakts) bzw. TGF-ß (10 ng/ml, Positivkontrolle) relativ zum Gesamtproteingehalt der Zellen.
Abbildung 5: Prokollagen Proteingehalt in humanen dermalen Fibroblasten 24 h nach Applikation von reinem Medium (Vehikel) bzw. 100 ppm (= 100 µg/ml) *Cyanidium caldarium* Extrakt (Konzentration bezogen auf Trockenmasse des Extrakts) relativ zum Gesamtproteingehalt der Zellen.
Abbildung 6: Genexpression von IL1-α in SkinEthic Hautmodellen 24 h nach UVA Bestrahlung und Applikation von 750 ppm (= 750 µg/ml) *Cyanidium caldarium* Extrakt (Konzentration bezogen auf Trockenmasse des Extrakts) bzw. Wasser (Vehikel) relativ zur Genexpression von GAPDH.
Abbildung 7: Genexpression von TNF-α in SkinEthic Hautmodellen 24 h nach UVA Bestrahlung und Applikation von 750 ppm (= 750 µg/ml) *Cyanidium caldarium* Extrakt (Konzentration bezogen auf Trockenmasse des Extrakts) bzw. Wasser (Vehikel) relativ zur Genexpression von GAPDH.
Abbildung 8: Genexpression von NFκ-B in SkinEthic Hautmodellen 24 h nach UVA Bestrahlung und Applikation von 750 ppm (= 750 µg/ml) *Cyanidium caldarium* Extrakt (Konzentration bezogen auf Trockenmasse des Extrakts) bzw. Wasser (Vehikel) relativ zur Genexpression von GAPDH.
Abbildung 9: Interleukin 1-alpha Proteingehalt in SkinEthic Hautmodellen 24 h nach UVA Bestrahlung und Applikation von 750 ppm (= 750 µg/ml) *Cyanidium caldarium* Extrakt (Konzentration bezogen auf Trockenmasse des Extrakts) bzw. Wasser (Vehikel) relativ zum Gesamtproteingehalt der Zellen.
Abbildung 10: Effekt von *Cyanidium caldarium* Extrakt auf die Koloniebildung von epidermalen Progenitor-Zellen. CFE nach Applikation von 100 ppm (= 100 µg/ml) *Cyanidium caldarium* Extrakt (Konzentration bezogen auf Trockenmasse des Extrakts) bzw. reinem Medium (Vehikel).
Abbildung 11: Genexpression verschiedener Gene in epidermalen Progenitor-Zellen nach Applikation von 100 ppm (= 100 µg/ml) *Cyanidium caldarium* Extrakt (Konzentration bezogen auf Trockenmasse des Extrakts) bzw. reinem Medium (Vehikel) relativ zur Genexpression von B2M.
Abbildung 12: Mittelwerte des Parameters E-Modul vor und nach Behandlung der Haarproben mit Leave-On Haarkonditionierer.
Abbildung 13: Hautelastizitätsparameter R1. Relative Veränderung bezogen auf Vehikelformulierung nach acht Wochen Behandlung mit 1% und 5% *Cyanidium caldarium* Extrakt.
Abbildung 14: Hautelastizitätsparameter R4. Relative Veränderung bezogen auf Vehikelformulierung nach acht Wochen Behandlung mit 1% und 5% *Cyanidium caldarium* Extrakt.
Abbildung 15: Haut Texturparameter. Relative Veränderung bezogen auf Vehikelformulierung nach acht Wochen Behandlung mit 1% und 5% *Cyanidium caldarium* Extrakt.
Abbildung 16: Haut Oberfläche und Volumenparameter. Relative Veränderung bezogen auf Vehikelformulierung nach acht Wochen Behandlung mit 1% und 5% *Cyanidium caldarium* Extrakt.

### Beispiele:

### Beispiel 1: Anzucht von Cyanidium caldarium

Die präparative Gewinnung von *Cyanidium caldarium* Biomasse für die Extraktherstellung erfolgt in geschlossenen Photobioreaktoren wie in DE4411486, DE29607285, WO9105849, WO2010/149154 oder DE102009028474A1 (WO2011/018082A2) detailliert beschrieben. Das Kultivierungsverfahren wurde an die spezifischen Wachstumsbedingungen der Rotalge *Cyanidium caldarium* (SAG 16.91) angepasst. Unter Minimierung des Scherstresses durch Auswahl geeigneter Pumpenkonfigurationen, kontinuierlicher Lichtadaptation durch variable Gestaltung des Lichtregimes während der Kultivierung von 50 bis 2000 uE*m⁻²s⁻¹ und Einstellung des pH-Wertes von <3 können sowohl hohe Wachstumsdichten als auch reproduzierbare Biomassequalitäten erzielt werden.

Das verwendete Kulturmedium bestand pro Liter destilliertem Wasser aus 4.5 g (NH₄)₂SO₄, 0.6 g MgSO₄ x 7 H₂O, 0.6 g KH₂PO₄, 0.03 g CaCl₂ x 2 H₂O, 0.01 g FeSO₄, 0.018 g Ethylendiamintetraessigsäure-Dinatrium-Dihydrat, 0.005 g H₃BO₃, 0.004 MnCl₂, 0.00068 g ZnSO₄ x 7 H₂O, 0.00052 g Na₂MoO₄ x 2 H₂O, 0.00008 g CuSO₄ x 5 H₂O, 0.00008 g NaVO₃ x 4 H₂O, 0.00064 g CoCl₂ x 6 H₂O. Der pH-Wert des Mediums wurde durch Zugabe von 1 N H₂SO₄ bzw. durch Regulierung der CO₂-Konzentration auf 2-3 eingestellt.

Für eine Vorkultur wurde ein 5 L Erlenmeyerkolben mit 1 L Medium befüllt und mit *Cyanidium caldarium* (SAG 16.91) Stammkultur beimpft. Unter leichtem Schütteln wurde bei 30 °C unter Leuchstoffröhren mit 140 µE*m⁻²s⁻¹ inkubiert. Das Wachstum der Algenkultur wurde durch Absorption bei 550 nm kontrolliert. Sobald das Wachstum die exponentielle Phase erreichte (OD 1.0), wurden die Zellen mittels Zentrifugation bei 5000 rpm für 10 Minuten geerntet und die Pelltets mit destilliertem Wasser gewaschen. Die feuchte Biomasse wurde bei -20 °C bis zur weiteren Verarbeitung gelagert.

### Beispiel 2: Wässrige Extraktion von Cyanidium caldarium

Die Ernte aus Photobioreaktoren erfolgt wie zum Beispiel in DE10136645 (EP 1277831) oder WO9105849 beschrieben.

Die Anwendung von Ultraschall zum Aufschluss der Zellen von Mikroalgen wurde beschrieben und ist unter anderem anhand von US2009069213, Beispiel 3 und US2008299147, Beispiel 3 nachvollziehbar. Die Abtrennung der unlöslichen Bestandteile erfolgte durch Filtration mit Beutelfilter 200 µm. Zur Konservierung wird dem wässrigen Extrakt 1.0 % Rokonsal BS zugesetzt. Die Bestimmung der Trockenmasse (105 °C) des wässrigen *Cyanidium caldarium*-Extrakts mittels Sartorius MA30 Moisture Analyzer ergab einen Wert von 2.5 %.

### Beispiel 3: Nachweis von GABA in Cyanidium caldarium

Der Nachweis von GABA erfolgte durch Hochleistunsflüssigkeitschromatografie (HPLC) nach Derivatisierung mittels *ortho*-Phthaldialdehyd (OPA). Das HPLC-Gerät bestand aus Jasco PU 2080Plus Pumpe, Jasco AS 2055Plus Autosampler, Jasco FP-2020Plus Fluoreszenzdetektor und Jasco ChromPass 1.8.6.1 Integrator (Jasco Germany GmbH, Gross-Umstadt, Deutschland). Bei der verwendeten stationären Phase handelte es sich um eine Zorbax Eclipse XDB-C18, 4.6 x 150 mm, 3.5 µm Säule (Agilent Technologies, Inc.) bei einer Temperatur von 40 °C. Als mobile Phase wurde folgender Gradient (A: 2 % Acetonitril, 2 % Tetrahydrofuran, 96 % 50 mM Phosphorsäure mit NaOH auf pH 7.5); B: 65 % Acetonitril, 35 % Wasser) verwendet: 0-2 min 20 % B konstant, 2-8 min 20-40 % B linear, 8-11 min 40-100 % B linear, 11-38 min 100 % B konstant. Die Flussrate betrug 1.5 mL/min. Injiziert wurden 5 µL des Analyten mit 15 µL des OPA-Reagenzes (1 mL Phthaldialdehyd-Reagenz, Solution Complete, Sigma-Aldrich P0532 und 0.5 µL Mercaptoethanol). Die Extinktionswellenlänge betrug 340 nm, die Emissionswellenlänge 455 nm. Als interner Standard diente 1,6-Diaminohexan. Die Vorbereitung des Analyten erfolgte durch Verdünnung von 1 mL des wässrigen *Cyanidium caldarium*-Extraktes mit Wasser auf 100 mL.

Ein typischer Messwerte für GABA im *Cyanidium caldarium*-Extrakt lag bei 0,090 %. Dieser Wert entsprach 3,3 % GABA bezogen auf *Cyanidium caldarium*-Trockenmasse.

### Beispiel 4: Genexpressions-Analyse von Cyanidium caldarium Extrakt auf humanen dermalen Fibroblasten mittels Gen-Chip-Studie

### Methode:

Im vorliegenden Beispiel wurde die Wirkung von *Cyanidium caldarium* Extrakt auf die Genexpression in Fibroblasten (normal human dermal fibroblasts, NHDFs) untersucht. Dazu wurden zunächst primäre humane dermale Fibroblasten (human dermal fibroblasts derived from neonatal skin (HDF), cryokenserviert, Lifeline Cell Technology, bezogen über CellSystems^{®} Biotechnologie Vertrieb GmbH, St. Katharinen, D) in Minimum Essential Medium (MEM) mit Earle's Salzen (EMEM) (PAA Laboratories GmbH, Pasching, A) unter Zusatz von 10 % fetalem Kälberserum (FBS - fetal bovine serum, (Invitrogen Ltd, UK), 1 % nicht essentieller Aminosäuren (NEAA (100x) - non essential amino acids, PAA, Pasching, A), 1 % L-Glutamin (100x) (Invitrogen Ltd, UK) und 1 % Penicillin/Streptomycin (5000U/mL Penicillin und 5000 µg/mL Streptomycin, Invitrogen Ltd, UK) bei 37 °C und 5% CO₂ kultiviert. Für Genexpressionsstudien wurden die Zellen in 6-Well-Platten ausgesät und bis zur Subkonfluenz (maximal 60%) kultiviert.

Anschliessend wurde das Medium von den Zellen abgenommen und durch frisches Medium mit *Cyanidium caldarium* Extrakt ersetzt. Endkonzentration von *Cyanidium caldarium* Extrakt im Medium war 100 ppm (=100 µg/ml, bezogen auf *Cyanidium caldarium*-Extrakt Trockenmasse). Als Kontrolle wurde die Kultivierung ohne Wirkstoff, nur mit Medium (Vehikel) durchgeführt. Sämtliche Kultivierungen wurden dreifach vorgenommen (3 biologische Replikate).

Nach 24-stündiger Kultivierung wurde das Medium abgenommen und die Zellen durch Zugabe von RNeasy Lysis Buffer (Qiagen, Hilden, D) lysiert. Die Isolation der Gesamt-RNA erfolgte nach Herstellerangaben. Zusammengefasst wurde die Gesamt-RNA mittels RNeasy Mini Kit (Qiagen, Hilden, D) isoliert. Die RNA-Qualität wurde mittels Agilent 2100 Bioanalyzer und Agilent RNA 6000 Nano Kit (Agilent Technologies, Inc., Santa Clara, CA, USA) bestimmt. Die drei biologischen Replikate wurden gepoolt und die Konzentration der gepoolten Proben wurde mittels photometrischer Messung bei 260/280 nm bestimmt.

Die Analyse der Genexpression erfolgte mittels Affymetrix GeneChip Expressionsanalysen inkl. Standard-Datenauswertung nach Herstellerangaben (Human Gene 1.0 ST Array, Expression Console Software, AFFYMETRIX, INC., Santa Clara, CA, USA).

Die Ergebnisse sind in Tabelle 1 gezeigt.

**Tabelle 1: Zusammenfassung mind. 2-fach hoch oder runter regulierter Gene in humanen dermalen Fibroblasten 24 h nach Applikation von 100 ppm (= 100 µg/ml) Cyanidium caldarium Extrakt (Konzentration bezogen auf Trockenmasse des Extrakts).**

| **Gen** | **Protein/Funktion** | **Regulation durch *Cyanidium*-Extrakt 100 ppm** |
|---|---|---|
| ELN | Elastin;neben Fibrillin Hauptkomponente elastischer Fasern | +2.4 |
| FBN2 | Fibrillin; neben Elastin Hauptkomponente elastischer Fasern | + 2.2 |
| COL3A1 | Kollagen Typ III Alpha-1 Kette; Kollagen Typ III ist ein fibrilläres Kollagen, daß in der Haut eng assoziiert mit dem Hauptkollagen Kollagen Typ I vorliegt. | + 1.8 |
| COL11A1 | Kollagen Typ XI Alpha-1 Kette | + 2.5 |
| LUM | Lumican; gehört zur Familie der kleinen Leucin-reichen Proteoglykane (SLRP; small leucine rich proteogylcan); interagiert mit Kollagen-Fibrillen und reguliert dadurch die interfibrillären Abstände | + 2.0 |
| ACAN | Aggrecan; großes aggregierendes Proteoglykan; wichtige strukturelle Komponente der extrazellulären Matrix | + 2.7 |
| SDC2 | Syndecan; ein Heparan-Sulfat Proteoglykan; wichtig für die Zell-Proliferation, Zell-Migration und Zell-Matrix Interaktionen | + 2.1 |
| CTGF | Connective tissue growth factor; zentraler Faktor für die Steuerung des Aufbaus der extrazellulären Matrix | +2.4 |
| SULF1 | Sulfatase; Entfernung von Sulfat-Resten von Heparan-Sulfat; Signaltransduktion | + 2.7 |
| MMP1 | Matrix metalloprotease 1; Abbau von Kollagen Typ I, II, III | -5.7 |
| MMP3 | Matrix metalloprotease 3; Abbau von Fibronectin, Laminin, Kollagen Typ III, IV, IX, X | -2.1 |

Die Gen-Chip-Studien in humanen dermalen Fibroblasten zeigten eine gleichmäßige Stimulation von Elastin (ELN) und Fibrillin (FBN2), den wichtigsten Strukturkomponenten von elastischen Fasern, durch die Zugabe von *Cyanidium caldarium* Extrakt. Zudem wurde die Genexpression von wichtigen Strukturproteinen des Bindegewebes, Kollagen III und XI (COL3A1, COL11A1), gesteigert. Andere Proteine wie Aggrecan (ACAN), Syndecan (SDC2) und Lumican (LUM) haben gerüstgebende Funktion oder helfen bei organisierenden Wechselwirkungen zwischen verschiedenen extrazellulären Matrix (ECM = extracellular matrix) Komponenten und Fibroblastenzellen. Auch die Gene dieser Proteine (LUM, ACAN, SDC2) wurden durch *Cyanidium caldarium* Extrakt hochreguliert. Der Abbau von extrazellulären Matrix Komponenten wurde effizient durch Herunterregulation von Matrix-Metalloprotease 1 (MMP-1) und Matrix-Metalloprotease 3 (MMP-3) verhindert. Zudem ist *Cyanidium caldarium* Extrakt ein potentieller Mediator des Bindegewebs-Wachstumsfaktors (connective tissue growth factor; CTGF).
Zusammenfassend zeigt die beschriebene Genregulierung, dass das erfindungsgemäße Kosmetikum vielmehr die Gesamtstruktur der ECM als nur die der einzelnen Komponenten der ECM stärkt.

### Beispiel 5: Gen- und Proteinexpressions Analyse von Cyanidium caldarium Extrakt auf humanen dermalen Fibroblasten mittels qRT-PCR und ELISA

### Methode:

Im vorliegenden Beispiel wurde die Wirkung von *Cyanidium caldarium* Extrakt auf die Gen- und Proteinexpression in Fibroblasten (normal human dermal fibroblasts, NHDFs) untersucht.

Dazu wurden zunächst primäre humane dermale Fibroblasten (human dermal fibroblasts derived from neonatal skin (HDF), cryokenserviert, Lifeline Cell Technology, bezogen über CellSystems® Biotechnologie Vertrieb GmbH, St. Katharinen, D) in Minimum Essential Medium (MEM) mit Earle's Salzen (EMEM) (PAA Laboratories GmbH, Pasching, A) unter Zusatz von 10 % fetalem Kälberserum (FBS - fetal bovine serum, Invitrogen Ltd, UK), 1 % nicht essentieller Aminosäuren (NEAA (100x) - non essential amino acids, PAA, Pasching, A), 1 % L-Glutamin (100x) (Invitrogen Ltd, UK) und 1 % Penicillin/Streptomycin (5000U/mL Penicillin und 5000 µg/mL Streptomycin/mL, Invitrogen Ltd, UK) bei 37 °C und 5% CO2 bis zum Erreichen einer ausreichenden Zellzahl kultiviert. Für Genexpressionsstudien wurden die Zellen in 6-Well-Platten ausgesät und bis zur Subkonfluenz (maximal 60%) kultiviert.

Anschließend wurde das Medium von den Zellen abgenommen und durch frisches Medium mit *Cyanidium caldarium* Extrakt ersetzt. Endkonzentration von *Cyanidium caldarium* Extrakt im Medium war 100 ppm (=100 µg/ml, bezogen auf Trockenmasse des Extrakts). Als Kontrolle wurde die Kultivierung ohne Wirkstoff, nur mit Medium (Vehikel) bzw. mit 10 ng/ml Transforming growth factor beta 3 (TGFß-3) als Positivkontrolle durchgeführt. Sämtliche Kultivierungen wurden dreifach vorgenommen (3 biologische Replikate).

Für die Untersuchung der Genexpression wurde nach 24-stündiger Kultivierung das Medium abgenommen und die Zellen durch Zugabe von RNeasy Lysis Buffer (Qiagen, Hilden, D) lysiert. Die Isolation der Gesamt-RNA erfolgte nach Herstellerangaben. Zusammengefasst wurde die Gesamt-RNA mittels RNeasy Mini Kit (Qiagen, Hilden, D) isoliert. Die RNA-Qualität und -Quantität wurde mittels Agilent 2100 Bioanalyzer und Agilent RNA 6000 Nano Kit (Agilent Technologies, Inc., Santa Clara, CA, USA) bestimmt.

Von jeder Probe wurde die Genexpression von Kollagen (COL1A1), Elastin (ELN), Matrixmetalloproteinase-1 (MMP-1), Fibrillin-2 (FBN2), Connective tissue growth factor (CTGF), Syndecan-2 (SDC2) und Lumican (LUM) mittels quantitativer real-time Polymerase Kettenreaktion (qRT-PCR) untersucht. Dazu wurden je 100 ng Gesamt-RNA für die cDNA-Synthese eingesetzt, wobei der Super Script III First Strand Synthesis Super Mix (Invitrogen Ltd, UK) nach Herstellerangaben eingesetzt wurde. Für die PCR-Reaktion wurde das Quanti Tect SYBR Green PCR Kit (Qiagen, Hilden, D) entsprechend der Herstellerangaben eingesetzt. Neben der-Expression der Zielgene wurde auch die Expression des Glycerinaldehyd-3-phosphat-Dehydrogenase (GAPDH)-Gens als Referenz quantitativ bestimmt. Für die Zielgene wurden jeweils folgende Primer-Paare eingesetzt:
**COL1A1: 60 °C**
   5'-GCCTCGGAGGAAACTTTG-3' Seq. ID Nr. 01
   5'-GACCCATGGGACCTGAAG-3' Seq. ID Nr. 02
**ELN: 56 °C**
   5'-GCCCAGTTTGGCCTAGTG-3' Seq. ID Nr. 03
   5'-CAGCAGCACCGTATTTAG-3' Seq. ID Nr. 04
**MMP-1: 60 °C**
   5'-TGGGAGCAAACACATCTGA-3' Seq. ID Nr. 05
   5'-ATCACTTCTCCCCGAATCGT-3' Seq. ID Nr. 06
**FBN2: 60 °C**
   5'-TCGCCCGGCAGCAAACTCAG-3' Seq. ID Nr. 07
   5'-TCACACCGCTCACAGGGGCT-3' Seq. ID Nr. 08
**CTGF: 60 °C**
   5'-CAGGCTAGAGAAGCAGAGCC-3' Seq. ID Nr. 09
   5'-TGGAGATTTTGGGAGTACGG-3' Seq. ID Nr. 10
**SDC2: 60 °C**
   5'-CGTGGATCCTGCTCACCT-3' Seq. ID Nr. 11
   5'-CAATGGAGCTGTTGTCAAGG-3' Seq. ID Nr. 12
**LUM: 56 °C**
   5'-GGTTGAGCTGGATCTGTCCT-3' Seq. ID Nr. 13
   5'-AGTAGGATAATGGCCCCAGG-3' Seq. ID Nr. 14
**GAPDH: 60°C**
   5'-ACCACAGTCCATGCCATCAC-3' Seq. ID Nr. 15
   5'-TCCACCACCCTGTTGCTGTA-3' Seq. ID Nr. 16

Die PCR-Reaktionen wurden auf einem Opticon 1 (MJ Research, Waltham, MA, USA) durchgeführt. Für sämtliche PCRs wurden Doppelbestimmungen der biologischen Triplikate durchgeführt, und für die Auswertung wurden Mittelwerte aller Messungen kalkuliert. Das PCR-Protokoll wies folgendes Profil auf: Schritt 1), 15 Minuten Aktivierung der Hot Start Polymerase bei 95 °C; Schritt 2), 15 Sekunden Denaturierung bei 94 °C; Schritt 3), 30 Sekunden Primer-Annealing bei 56°C bzw. 60 °C je nach Primer-Paar; Schritt 4), 30 Sekunden Extension bei 72 °C. Die Zyklenzahl der Schritte 2)-4) lag bei 44. Für den relativen Vergleich der Genexpression wurde die 2(-delta delta C(T))-Methode angewandt (Livak KJ, and Schmittgen TD: Analysis of relative gene expression data using real time quantitative PCR and the 2 (-delta delta C(T)) method. Methods 2001, 25: 402 - 408).

Für die Untersuchung der Proteinexpression wurde nach 24-stündiger Kultivierung das Medium abgenommen und die Zellen mittels Ultraschall (10 Sekunden bei 34-40 kHz) lysiert.

Der Gesamtproteingehalt der Ultraschall-Lysate wurde mittels Bradford-Test (Bio-Rad Protein Assay, Bio-Rad Laboratories, Inc., Hercules, CA, USA) nach Herstellerangaben bestimmt. Von jeder Probe wurde mittels ELISA-Technik der Proteingehalt von Elastin im Zell-Extrakt (human Elastin ELISA Kit, ABO Swiss Co., Ltd, Fujian, CN) und Lumican (human Lumican ELISA Kit, ABO Swiss Co., Ltd, Fujian, CN) bzw. Prokollagen (Procollagen Type I C-Peptide (PIP) EIA Kit, TAKARA BIO INC., JP) im Kulturüberstand nach den jeweiligen Herstellerangaben gemessen. Die absolute Proteinmenge wurde relativ zum Gesamtprotein bestimmt.

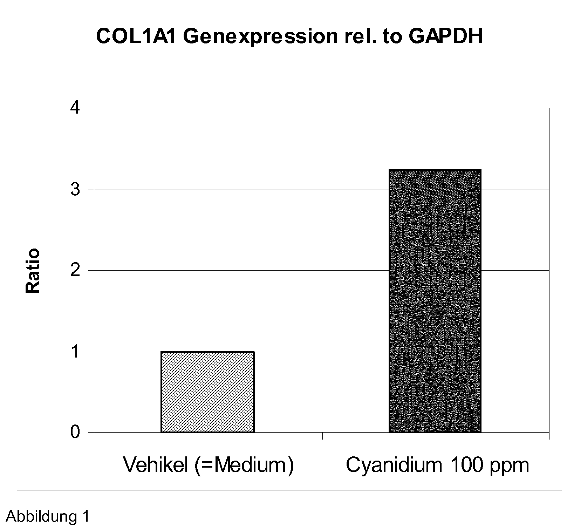

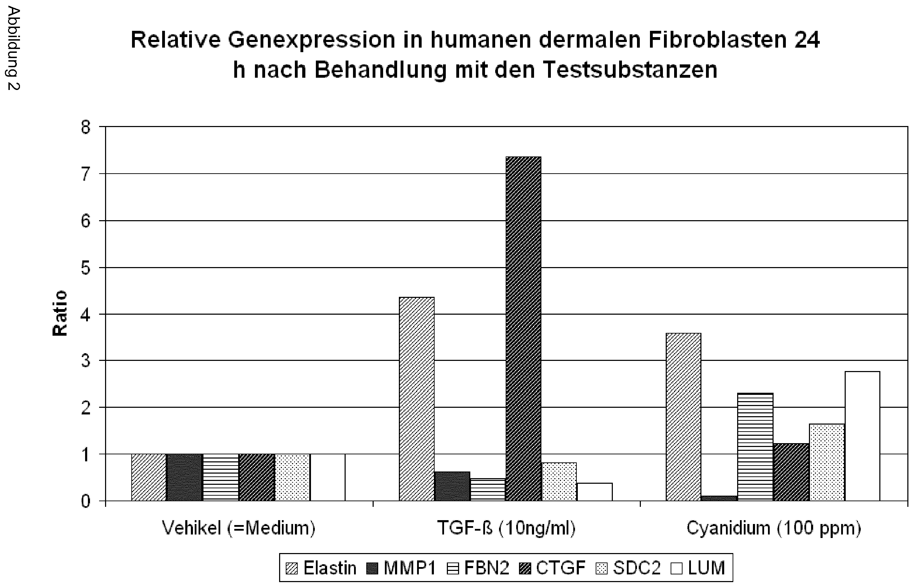

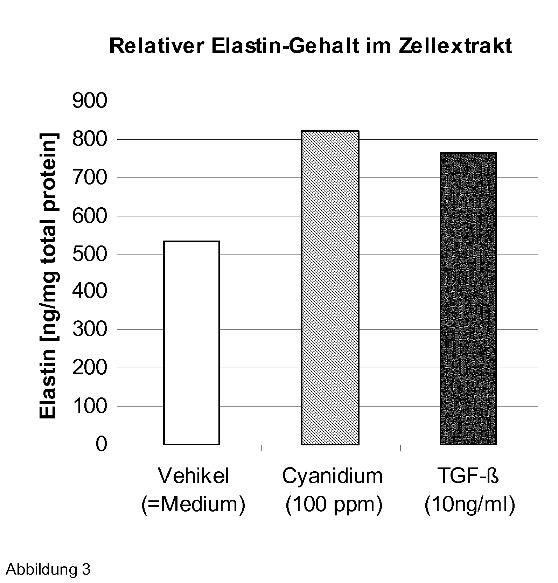

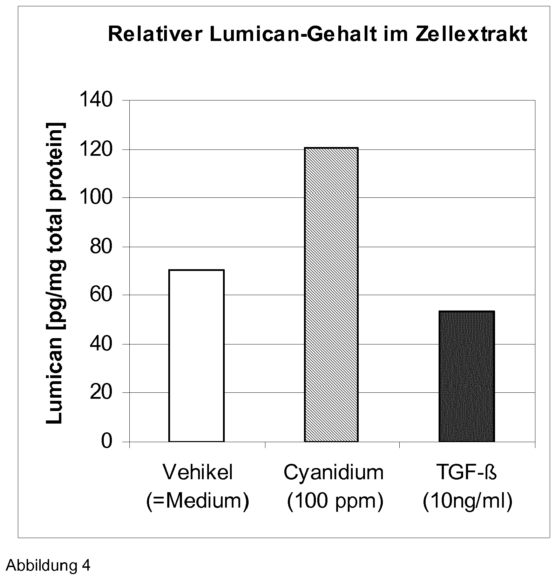

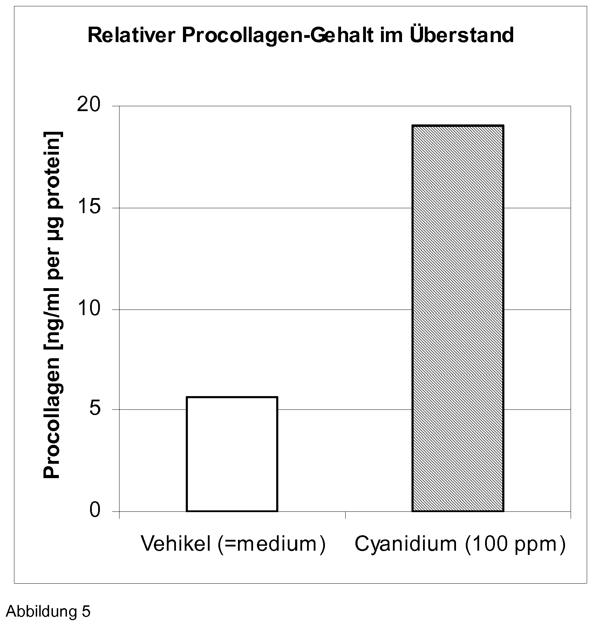

Die Ergebnisse sind in den Abbildungen 1 bis 5 dargestellt und zeigen, dass die Behandlung der Keratinozyten mit *Cyanidium caldarium* Extrakt die Genexpression von COL1A1, ELN, FBN2, SDC2 und LUM signifikant stimuliert. Die Expression des MMP-1 Gens wurde signifikant runter reguliert. Sowohl Elastin als auch Lumican und Prokollagen wurden außerdem nach 24-stündiger Behandlung der Zellen mit *Cyanidium caldarium* Extrakt auf Proteinebene positiv beeinflusst.

Zusammenfassend bestätigt die beschriebene Genregulation die Ergebnisse der Gen-Chip Analyse. Das erfindungsgemäße Kosmetikum stärkt vielmehr die Gesamtstruktur der ECM als nur deren einzelne Komponenten.

### Beispiel 6: Einfluss von Cyanidium caldarium Extrakt auf UVA-gestresste humane Epidermis-Modelle

### Methode:

Im vorliegenden Beispiel wurde die Wirkung von *Cyanidium caldarium* Extrakt auf die Gen- und Protein-Expression in Epidermis Hautmodellen (reconstituted human epidermis) untersucht.
Dazu wurden Hautmodelle (reconstructed epidermis of normal human keratinocytes, RHE/S/17, SkinEthic laboratories, Nice, F) in Maintenance Medium (SkinEthic laboratories, Nice, F) bei 37 °C und 5% CO₂ für 24 Stunden kultiviert. Für Gen- und Protein-Expressionsstudien wurden die Hautmodelle anschließend topisch mit je 50 µl *Cyanidium caldarium* Extrakt bzw. den entsprechenden Kontrollen behandelt. Nach 1 Stunde wurde der Überschuss der Proben abgetupft. Endkonzentration von *Cyanidium caldarium* Extrakt im Medium war 750 ppm (= 750 µg/ml, bezogen auf Trockenmasse des Extrakts). Als Kontrolle wurde die Kultivierung ohne Wirkstoff bzw. nur mit Wasser (Vehikel) durchgeführt. Sämtliche Kultivierungen wurden dreifach vorgenommen (3 biologische Replikate).
Nach 24-stündiger Kultivierung wurden die Hautmodelle mit einer Dosis von 40 J/cm² bei 9,5 mW/cm² UVA bestrahlt. Anschließend erfolgten ein Medienwechsel und eine erneute Applikation der Prüfsubstanzen bzw. Kontrollen. 24 Stunden nach der zweiten Applikation wurden die Hautmodelle in RNAlater (RNA Stabilization Reagent, Qiagen, Hilden, D) überführt. Der Unterstand der Hautmodelle (Medium) wurde zur weiteren Analyse der Proteinexpression unter Zugabe von Complete Protease Inhibitor Cocktail Tablets (F. Hoffmann-La Roche Ltd, Basel, CH) eingefroren (-80 °C).
Die Isolation der Gesamt-RNA aus den Hautmodellen erfolgte mittels RNeasy Lipid Tissue Mini Kit (Qiagen, Hilden, D) nach Herstellerangaben. Die RNA-Qualität und - Quantität wurde mittels Agilent 2100 Bioanalyzer und Agilent RNA 6000 Nano Kit (Agilent Technologies, Inc., Santa Clara, CA, USA) bestimmt.

Von jeder Probe wurde die Genexpression von Interleukin 1-alpha (IL1-α), Tumor necrosis factor-alpha (TNF-α) und Nuclear factor kappa-light-chain-enhancer of activated B cells (NFκ-B) mittels quantitativer real-time Polymerase Kettenreaktion (qRT-PCR) untersucht. Dazu wurden je 100 ng Gesamt-RNA für die cDNA-Synthese eingesetzt, wobei der Super Script III First Strand Synthesis Super Mix (Invitrogen Ltd, UK) nach Herstellerangaben eingesetzt wurde. Für die PCR-Reaktion wurde das Quanti Tect SYBR Green PCR Kit (Qiagen, Hilden, D) entsprechend der Herstellerangaben eingesetzt. Als Referenzgen wurde auch die Expression von Glycerinaldehyd-3-phosphat-Dehydrogenase (GAPDH) quantitativ bestimmt. Es wurden jeweils folgende Primer-Paare eingesetzt:
**NFκ-B:**
   5'-CCTGGATGACTCTTGGGAAA-3' Seq. ID Nr. 17
   5'-TCAGCCAGCTGTTTCATGTC-3' Seq. ID Nr. 18
**TNF-α:**
   5'-CTCTGGCCCAGGCAGTCAGA-3' Seq. ID Nr. 19
   5'-GGCGTTTGGGAAGGTTGGAT-3' Seq. ID Nr. 20
**IL1-α:**
   5'-CACTCCATGAAGGCTGCATGG-3' Seq. ID Nr. 21
   5'-ACCCAGTAGTCTTGCTTTGTGG-3' Seq. ID Nr. 22
**GAPDH:**
   5'-ACCACAGTCCATGCCATCAC-3' Seq. ID Nr. 23
   5'-TCCACCACCCTGTTGCTGTA-3' Seq. ID Nr. 24

Die PCR-Reaktionen wurden auf einem Opticon 1 (MJ Research, Waltham, MA, USA) durchgeführt. Für sämtliche PCRs wurden Doppelbestimmungen der biologischen Triplikate durchgeführt, und für die Auswertung wurden Mittelwerte aller Messungen kalkuliert. Das PCR-Protokoll wies folgendes Profil auf: Schritt 1), 15 Minuten Aktivierung der Hot Start Polymerase bei 95 °C; Schritt 2), 15 Sekunden Denaturierung bei 94 °C; Schritt 3), 30 Sekunden Primer-Annealing bei 60 °C; Schritt 4), 30 Sekunden Extension bei 72 °C. Die Zyklenzahl der Schritte 2)-4) lag bei 44. Für den relativen Vergleich der Genexpression wurde die 2(-delta delta C(T))-Methode angewandt (Livak KJ, and Schmittgen TD: Analysis of relative gene expression data using real time quantitative PCR and the 2 (-delta delta C(T)) method. Methods 2001, 25: 402 - 408).

Für die Untersuchung der Proteinexpression wurde der Unterstand der Hautmodelle abgenommen und das Gesamtprotein aus den Hautmodellen als Kombinationsprotokoll zusätzlich zur RNA mittels RNeasy Lipid Tissue Mini Kit (Qiagen, Hilden, D) isoliert. Im Detail wurde aus den Proben der RNA Isolation die restliche Wasser-Phase entfernt. Es erfolgte die Zugabe von 0,3 ml 100 % Ethanol, die Proben wurden invertiert und für 2-3 min bei Raumtemperatur (RT) inkubiert. Anschließend wurden die Proben bei 4°C für 2 min bei 2000 x g zentrifugiert. Der Überstand mit den Proteinen wurde in ein neues Tube (2ml) gegeben. Durch Zugabe von 1,5 ml Isopropanol wurden die Proteine ausgefällt und für 10 min bei RT inkubiert. Anschließend wurden die Proben bei 4°C für 10 min bei 12000 x g zentrifugiert. Das Pellet wurde in 2ml Guanidin/Ethanol Lösung aufgenommen und 20 min bei RT inkubiert. Anschließend wurden die Proben bei RT für 5 min bei 7500 x g zentrifugiert. Dieser Waschschritt wurde 2 mal wiederholt. Nach Zugabe von 2 ml Ethanol (100%) wurden die Proben für 20 min bei RT inkubiert. Anschließend wurden die Proben bei RT für 5 min bei 7500 x g zentrifugiert. Das Pellet wurde 5-10 min an der Luft getrocknet, in 500 µl Urea/DTT Lösung aufgenommen und 1h bei RT inkubiert. Zur Lösung der Proteine wurden die Proben für 3 Minuten auf 95°C erhitzt.
Der Gesamtproteingehalt wurde mittels Bradford-Test (Bio-Rad Protein Assay, Bio-Rad Laboratories, Inc., Hercules, CA, USA) nach Herstellerangaben bestimmt. Von jeder Probe wurde mittels ELISA-Technik der Proteingehalt von Interleukin 1-alpha (IL1-α) (Human IL-1α ELISA Kit, (Invitrogen Ltd, UK) im Kulturüberstand nach den jeweiligen Herstellerangaben gemessen. Die absolute Proteinmenge wurde relativ zum Gesamtprotein bestimmt.

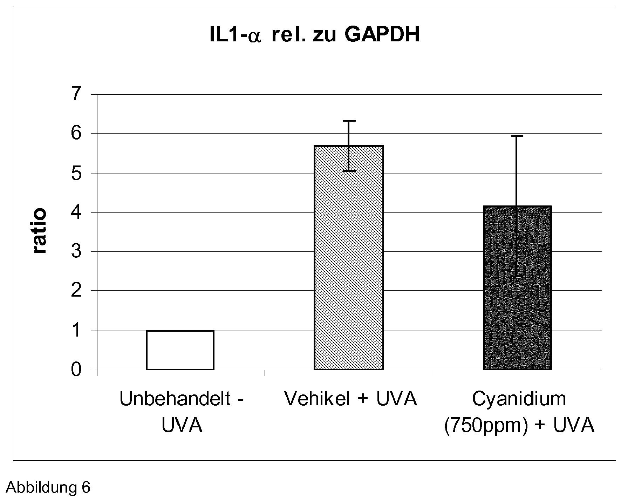

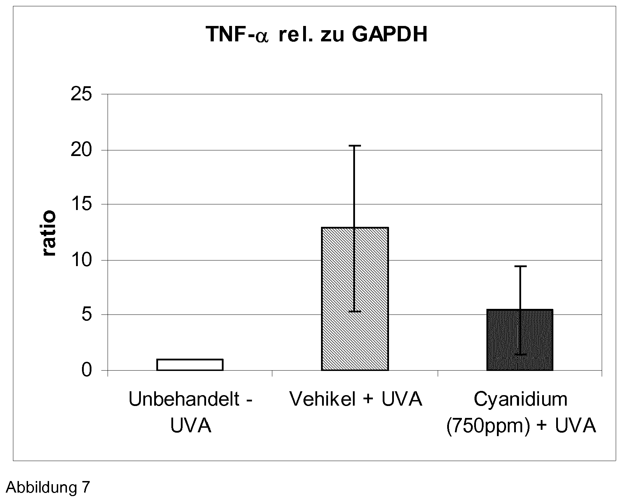

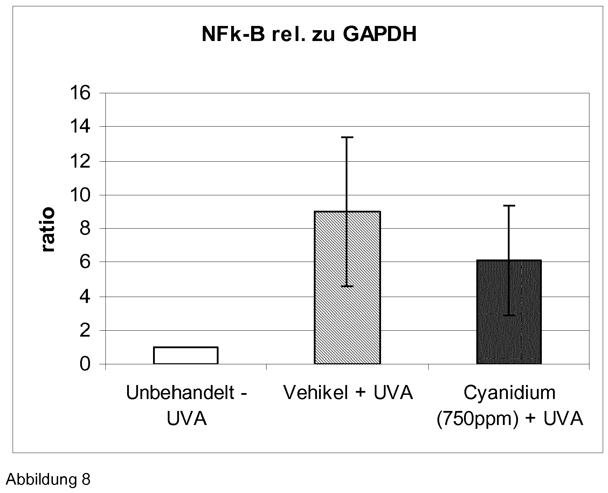

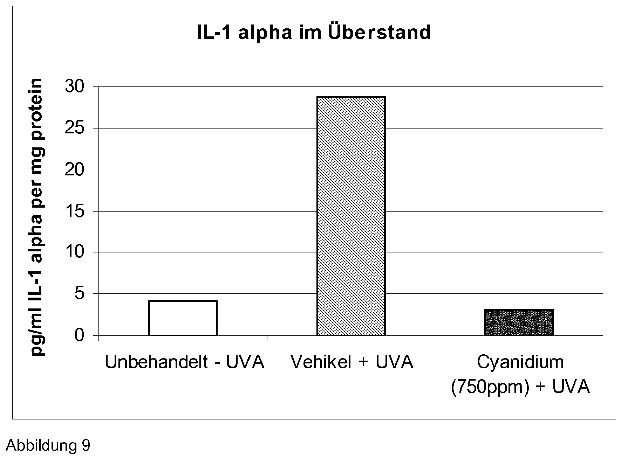

Die Ergebnisse dieser Studie sind in den Abbildungen 6 bis 9 dargestellt und zeigen, dass *Cyanidium caldarium* Extrakt die UVA-induzierte Expression von proinflammatorischen Markern auf molekularer Ebene reduzieren kann. *Cyanidium caldarium* Extrakt stellte somit die epidermale Homeostase an UVA-gestressten Hautmodellen wieder her, welche hauptsächlich durch NFκB sowie TNF-α und IL-1α reguliert wird.

### Beispiel 7: Einfluss von Cyanidium caldarium Extrakt auf epidermale Stammzellen

### Methode:

Im vorliegenden Beispiel wurde die Wirkung von *Cyanidium caldarium* Extrakt auf epidermale Stammzellen (Epidermal Keratinocyte Progenitors, HPEK) untersucht.

Dazu wurden zunächst CnT-57 NHEK Progenitor-Zellen (normal human epidermal keratinocyte progenitor cells (HPEKp05), aus neonataler Vorhaut abstammend, cryokonserviert bei P2, CELLnTEC Advanced Cell Systems AG, Bern, CH) in supplementiertem CnT-57 Medium (Progenitor Cell Targeted Epidermal Keratinocyte Medium based on low Bovine Pituitary Extract (BPE), CELLnTEC Advanced Cell Systems AG, Bern, CH) + 2 % Penicillin/Streptomycin Lösung (5000 U/mL Penicillin und 5000 µg/mL Streptomycin/mL, Invitrogen Ltd, UK) bei 37 °C und 5 % CO₂ bis zum Erreichen einer ausreichenden Zelldichte kultiviert.

Zur Bestimmung der Effizienz zur Koloniebildung (colony forming efficiency, CFE) wurden die Zellen anschließend bei geringer Aussaatdichte (800 Zellen/25 cm²) in Gegenwart von *Cyanidium caldarium* Extrakt für 8 Tage kultiviert.

Endkonzentration von *Cyanidium caldarium* Extrakt im Medium war 100 ppm (= 100 µg/ml, bezogen auf Algen-Trockenmasse). Als Kontrolle wurde die Kultivierung ohne Wirkstoff, nur mit Medium (Vehikel) durchgeführt. Sämtliche Kultivierungen wurden dreifach vorgenommen (3 biologische Replikate).

Nach Abnahme des Zellkulturmediums wurden die Zell-Kolonien mit Hämatoxylin/Eosin (CnT-ST-100 Stain Kit, CELLnTEC Advanced Cell Systems AG, Bern, CH) nach Herstellerangaben angefärbt. Die koloniebildenden Einheiten wurden mikroskopisch ausgezählt.

Für Genexpressionsstudien wurden die Zellen außerdem mit einer Aussaatdichte von 3000 Zellen/25 cm² in Gegenwart von *Cyanidium caldarium* Extrakt bis zur Subkonfluenz (75 %) für 7 Tage kultiviert.

Endkonzentration von *Cyanidium caldarium* Extrakt im Medium war 100 ppm (=100 µg/ml, bezogen auf Trockenmasse des Extrakts). Als Kontrolle wurde die Kultivierung ohne Wirkstoff, nur mit Medium (Vehikel) durchgeführt. Sämtliche Kultivierungen wurden dreifach vorgenommen (3 biologische Replikate).

Für die Untersuchung der Genexpression wurde das Medium abgenommen und die Zellen durch Zugabe von RNeasy Lysis Buffer (Qiagen, Hilden, D) lysiert. Die Isolation der Gesamt-RNA erfolgte nach Herstellerangaben. Zusammengefasst wurde die Gesamt-RNA mittels RNeasy Mini Kit (Qiagen, Hilden, D) isoliert. Die RNA-Qualität und -Quantität wurde mittels Agilent 2100 Bioanalyzer und Agilent RNA 6000 Nano Kit (Agilent Technologies, Inc., Santa Clara, CA, USA) bestimmt.

Von jeder Probe wurde die Genexpression von CD34 (hematopoietic progenitor cell antigen), CTNNB1 (ß-Catenine), ITGB1 (Integrin beta-1), ITGA6 (Integrin alpha-6), LRIG1 (Leucine-rich repeats and immunoglobulin-like domains protein 1), DSG3 (Desmoglein-3), MKI67 (Antigen KI-67) und KRT14 (Keratin-14) mittels quantitativer real-time Polymerase Kettenreaktion (qRT-PCR) untersucht. Dazu wurden je 100 ng Gesamt-RNA für die cDNA-Synthese eingesetzt, wobei das Superscript VILO cDNA Synthesis Kit (Invitrogen Ltd, UK) nach Herstellerangaben eingesetzt wurde. Für die PCR-Reaktion wurde das QuantiFast SYBR Green PCR Kit (Qiagen, Hilden, D) entsprechend der Herstellerangaben eingesetzt. Neben der Expression der Zielgene wurde auch die Expression des B2M (β2-Mikroglobulin)-Gens als Referenz quantitativ bestimmt. Für die Gene wurden jeweils folgende Primer-Paare eingesetzt:
**CD34:**
   5'-CCACCAGAGCTATTCCCAAAAG-3' Seq. ID Nr. 25
   5'-GCGGCGATTCATCAGGAAAT-3' Seq. ID Nr. 26
**CTNNB1:**
   5'-TGCCAAGTGGGTGGTATAGA-3' Seq. ID Nr. 27
   5'-TCAGATGACGAAGAGCACAGA-3' Seq. ID Nr. 28
**ITGB1:**
   5'-ATGTGAATGCCAAAGCGAAG-3' Seq. ID Nr. 29
   5'-CTACCAACACGCCCTTCATT-3' Seq. ID Nr. 30
**ITGA6:**
   5'-CTCTTCGGCTTCTCGCTG-3' Seq. ID Nr. 31
   5'-CCCGTTCTGTTGGCTCTCT-3' Seq. ID Nr. 32
**LRIG1:**
   5'-GACCTGCCCTCCTGGAC-3' Seq. ID Nr. 33
   5'-GTAGGTTCGGCAAGTCCTCA-3' Seq. ID Nr. 34
**DSG3:**
   5'-CGGCCTGCTGCTCCTTGGTC-3' Seq. ID Nr. 35
   5'-CACCGCTGTGCCTCTGGCAT-3' Seq. ID Nr. 36
**KI-67:**
   5'-ATCGTCCCAGGTGGAAGAGTT-3' Seq. ID Nr. 37
   5'-ATAGTAACCAGGCGTCTCGTGG-3' Seq. ID Nr. 38
**KRT14:**
   5'-GGCCTGCTGAGATCAAAGAC-3' Seq. ID Nr. 39
   5'-TCTGCAGAAGGACATTGGC-3' Seq. ID Nr. 40
**B2M:**
   5'-GTGCTCGCGCTACTCTCTCT-3' Seq. ID Nr. 41
   5'-TTCAATGTCGGATGGATGAA-3' Seq. ID Nr. 42

Die PCR-Reaktionen wurden auf einem StepOnePlus™ Real-Time PCR System (Life Technologies Corporation, CA, USA) durchgeführt. Für sämtliche PCRs wurden Doppelbestimmungen der biologischen Triplikate durchgeführt, und für die Auswertung wurden Mittelwerte aller Messungen kalkuliert. Das PCR-Protokoll wies folgendes Profil auf: Schritt 1), 5 Minuten Aktivierung der Hot Start Polymerase bei 95 °C; Schritt 2), 10 Sekunden Denaturierung bei 95 °C; Schritt 3), 30 Sekunden Primer-Annealing bei 60°C. Die Zyklenzahl der Schritte 2)+3) lag bei 40. Für den relativen Vergleich der Genexpression wurde die 2(-delta delta C(T))-Methode angewandt (Livak KJ, and Schmittgen TD: Analysis of relative gene expression data using real time quantitative PCR and the 2 (-delta delta C(T)) method. Methods 2001, 25: 402 - 408).

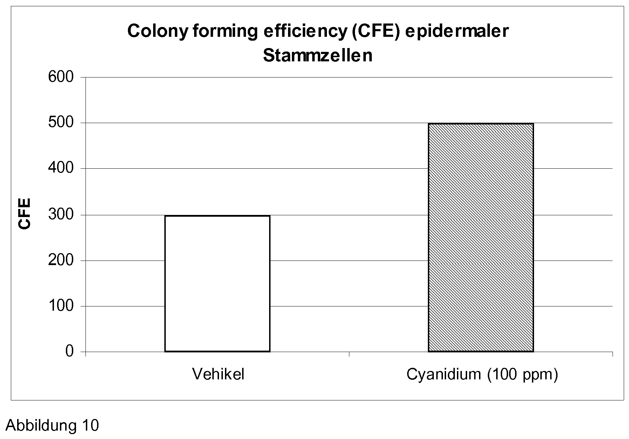

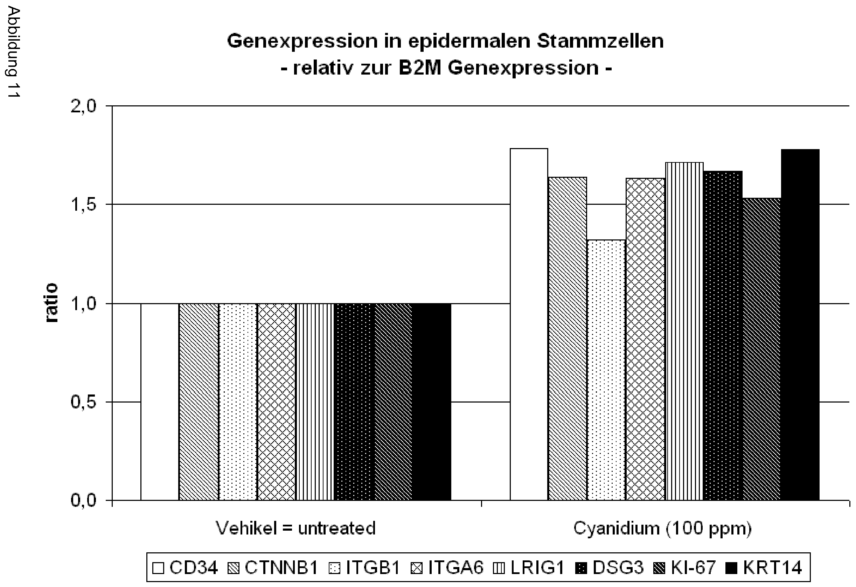

Die Ergebnisse dieser Studie sind in Abbildungen 10 und 11 dargestellt und zeigen, dass *Cyanidium caldarium* Extrakt die Effizienz von epidermalen Progenitor-Zellen zur Bildung von Kolonien während der Kultivierung signifikant steigern kann. Das bedeutet, dass *Cyanidium caldarium* Extrakt den Stammzellcharakter der Keratinozyten aufrecht erhält und sich die Zellen nicht in den Differenzierungs-Status begeben. Auf Genexpressionsebene war zu beobachten, dass beschriebene Stammzell-Markergene in den Progenitor-Keratinozyten hoch reguliert wurden, was ebenfalls ein Hinweis darauf ist, dass der Stammzellcharakter der Keratinozyten während der Kultivierung durch die Zugabe von *Cyanidium caldarium* Extrakt stimuliert werden kann.

Zusammenfassend zeigen die Beispiele 4 bis 7, dass *Cyanidium caldarium* Extrakt ein potentes Kosmetikum ist, um die Elastizität der Haut durch Aufbau von Elastin und den umgebenden extrazellulären dermalen Strukturen, wie Lumican und Kollagen, zu stärken. Desweiteren führt *Cyanidium caldarium* Extrakt bei Applikation auf UV gestresste Haut durch die anti-inflammatorische Wirkung dazu, dass die epidermale Homeostase der Haut wieder hergestellt wird. Zudem sorgt *Cyanidium caldarium* Extrakt für die Aufrechterhaltung der Stammzellfunktionen in der Epidermis und hilft somit drei der wichtigsten Funktionen als Schutz vor Hautalterung aufrechtzuerhalten.

### Beispiel 8: Verbesserung der mechanischen Eigenschaften von Haaren durch Behandlung mit Cyanidium caldarium Extrakt

Eine Tressenteil menschlicher Euro-Natur-Haare, remis, doppelt-gezogen mit einer Länge von 23 cm (21 cm freie Haare) und einer Breite von 2.0 cm mit einem Gewicht von 2.0 g und dunkelbrauner Farbe wurde unter dem laufenden Wasserhahn innerhalb von 10 Sekunden angefeuchtet. Anschließend wurden 8 g einer 30%igen wässrigen Wasserstoffperoxidlösung mit 4 g Basler Blond Claire Blondierpulver vermischt, 2 mL einer 25%igen wässrigen Ammoniaklösung hinzugegeben, erneut vermengt und 8 g der resultierenden Paste mit Hilfe eines Kamms und den geschützten Händen in das Tressenteil einmassiert. Nach einer Einwirkzeit von 30 Minuten bei Raumtemperatur wurde das Tressenteil für 2 Minuten unter laufendem Kranwasser von ca. 35 °C gewaschen und dann mittels eines elektronischen Haartrockners während 3 Minuten unter gleichzeitigem Kämmen getrocknet. Die gesamte Prozedur wurde ausgehend vom Anfeuchten des Tressenteils an einmal wiederholt.

Im Anschluss wurden 40 Haare aus dem Tressenteil entfernt und jeweils der mittlere Teil eines Haares mit einer Länge von 3 cm zwischen zwei Messinghülsen mit innenliegender Kunststoffbeschichtung verkrimpt. Die mittlere Fläche jedes einzelnen Haares wurde mittels Dia-stron FDAS760 Faserdimensionalsystem und UvWin PC Applikationssoftware vermessen. Die Haarproben wurden daraufhin in die Probenkassette eines Dia-stron Tensile Tester MTT 670 überführt und jeweils mit mittels Zitronensäure auf pH 7 eingestellten VE-Wasser versetzt. Eine Messung der einzelnen Haarsträhnen mit der Single Fiber Method (Extension 20%, Rate 20 mm/min, Gauge Force 2, Maximum Force 200, Break Threshold 5, Sample Size 30 mm) wurde gestartet. Danach wurden die Haarproben aus der Probenkassette entnommen und in einem Kunststoffschälchen mit VE-Wasser bedeckt. Nach 30 Minuten wurde das Wasser entfernt und die Haarproben mit Leave-On Haarkonditionierer (Tabelle 2) bedeckt.

**Tabelle 2. Testformulierungen. Angaben in Massenprozent. Zur Herstellung der Formulierungen wurden dem Fachmann bekannte, übliche Formulierungsverfahren eingesetzt.**

| | Rohstoff | Vehikel | 2.5% *Cyanidium caldarium* Extrakt |
|---|---|---|---|
| A | TEGINACID C^{®} (Ceteareth-25) | 0.5% | 0.5% |
| | TEGO Alkanol 1618 (Cetearyl Alcohol) | 2.0% | 2.0% |
| | *Cyanidium caldarium* Extrakt | | 2.5% |
| | Water | ad 100% | ad 100% |
| | Methyl chloro isothiazolinone, Methyl isothiazolinone (Kathon™ CG) | 0.05% | 0.05% |
| | Lactic Acid (10% in water) | pH 4.0-4.5 | pH 4.0-4.5 |

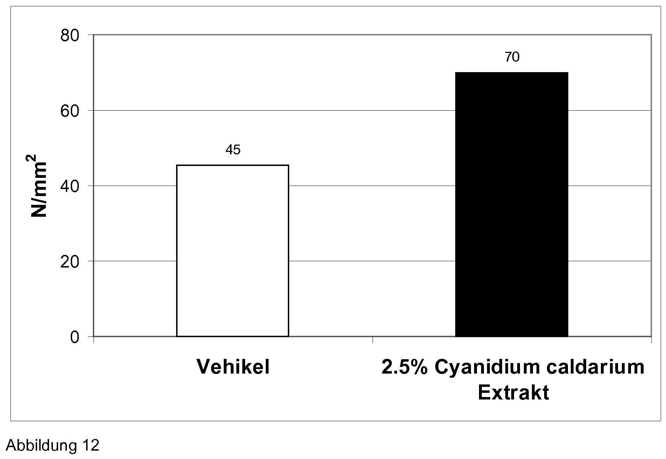

Nach einer Einwirkzeit von 30 Minuten wurde jede einzelne Haarproben unter dem laufenden Wasserstrahl von ca. 35 °C 6 Sekunden lang gewaschen. Die Haarproben wurden über Nacht bei 22 °C und 50% relativer Luftfeuchtigkeit getrocknet. Anschließend wurde die Messung der einzelnen Haarproben beginnend mit der Überführung in die Probenkassette des Dia-stron Tensile Tester MTT 670 wiederholt. Die Auswertung der Daten erfolgt mittels eines Tabellenkalkulationsprogramms anhand des Parameters Elastizitätsmodul (E-Modul; Abbildung 12). Der E-Modul beschreibt den Zusammenhang zwischen Spannung und Dehnung bei der Verformung eines festen Körpers bei linear elastischem Verhalten ("Hooke'scher Bereich"). Der Parameter E-Modul repräsentiert somit die Zugfestigkeit einer Haarfaser und wird in der Einheit N/mm² angegeben.

Wie die Ergebnisse der Zug-Dehnungsmessungen zeigen, wurde durch Zugabe von 2.5% *Cyanidium caldarium* Extrakt zum Leave-On Haarkonditionierer ein Anstieg des Parameters E-Modul um 25 N/mm² erzielt. Dadurch wurde gezeigt, dass die Zugfestigkeit von geschädigtem Haar durch *Cyanidium caldarium* Extrakt erhöht wird.

### Beispiel 9: Verstärkung der Hautelastizität und Verbesserung der Hautstruktur durch Cyanidium caldarium Extrakt

Um die Verstärkung der Hautelastizität und Verbesserung der Hautstruktur durch topische Applikation von *Cyanidium caldarium* Extrakt zu demonstrieren, wurde eine humane, randomisierte, Vehikel-kontrollierte, verblindete Applikationsüberwachung über einen Zeitraum von acht Wochen durchgeführt.

Das Panel umfasste Probanden im Alter von 33-59 Jahren (Mittelwert 47±8 Jahre). Zwanzig Probanden erhielten Vehikel-Formulierung, 19 Probanden die Vehikel-Formulierung enthaltend 1 % *Cyanidium caldarium* Extrakt und 21 Probanden die Vehikel-Formulierung enthaltend 5% *Cyanidium caldarium* Extrakt. Die Auftragung der Formulierungen erfolgte täglich zweimal (morgens und abends) auf der Innenseite des Unterarms. Appliziert wurden 2 Schübe aus einem Dosierfläschchen (0.25-0.30 g) durch Einmassieren. Die Zusammensetzung der Formulierung ist in Tabelle 3 gezeigt:

**Tabelle 3. Testformulierungen. Angaben in Massenprozent. Zur Herstellung der Formulierungen wurden dem Fachmann bekannte, übliche Formulierungsverfahren eingesetzt.**

| Rohstoff | Vehikel | 1% *Cyanidium* | 5% *Cyanidium* |
|---|---|---|---|
| TEGO^{®} Care 450 (Polyglyceryl-3 Methylglucose Distearate) | 3.0 | 3.0 | 3.0 |
| TEGO^{®} Alkanol 18 (Stearyl Alcohol) | 0.5 | 0.5 | 0.5 |
| TEGIN^{®} M Pellets (Glyceryl Stearate) | 1.0 | 1.0 | 1.0 |
| TEGOSOFT^{®} CT (Caprylic/Capric Triglycerides) | 6.0 | 6.0 | 6.0 |
| TEGOSOFT^{®} G 20 (Octyldodecanol) | 2.0 | 2.0 | 2.0 |
| TEGOSOFT^{®} PC 41 (Polyglyceryl-4 Caprate) | 2.0 | 2.0 | 2.0 |
| Water | 85.2 | 84.2 | 80.2 |
| Xantham Gum (Keltrol CG-SFT) | 0.3 | 0.3 | 0.3 |
| *Cyanidium caldarium* Extrakt | | 1.0 | 5.0 |
| Methylisothiazolinone, Ethylhexylglycerin (Euxyl K220) | 0.1 | 0.1 | 0.1 |
| pH (NaOH) | 5.5 | 5.5 | 5.5 |

Die Applikationsphase betrug acht Wochen. Gemessen wurde die Hautelastizität mittels Cutometer MPA 580 (Courage+Khazaka electronic GmbH) und die Beschaffenheit der Hautoberfläche mittels Visioscan VC 98 (Courage+Khazaka electronic GmbH) auf der Innenseite des Unterarms zum Zeitpunkt T0 vor der Applikationsphase und T8 nach acht Wochen. Registriert wurde der Mittelwert, der sich aus den Einzelwerten von mehreren Wiederholungsmessungen errechnet. Am Abend vor der jeweiligen Messung wurde keine Formulierung appliziert.

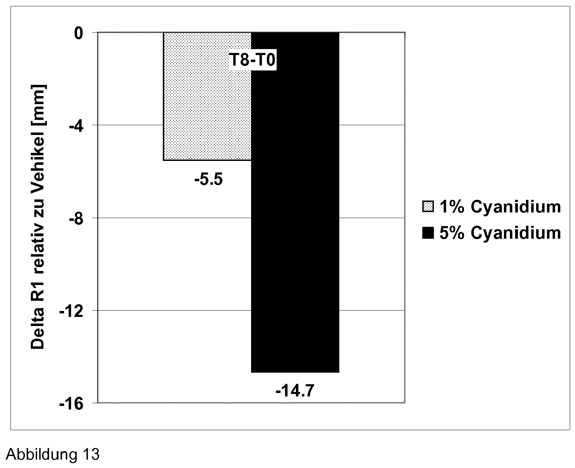

Wie die humane *in vivo* Studie zeigte, konnte über den Zeitraum von acht Wochen eine Abnahme des Hautelastizitätsparameter R1 beobachtet werden. R1 ist die verbleibende Deformierung der Haut nach dem ersten Dehnungszyklus, und repräsentiert die Fähigkeit der Haut, in ihren ursprünglichen Zustand zurückzukehren. Der Parameter R1 ist mit zunehmendem Alter stark erhöht. Die Abnahme von R1 betrug etwa 5 mm in der Gruppe der Personen, die 1 % *Cyanidium caldarium* Extrakt applizierten, während die Abnahme in der Gruppe der Personen, die 5% *Cyanidium caldarium* Extrakt applizierten, dreimal so stark ausgeprägt war (Abbildung 13).

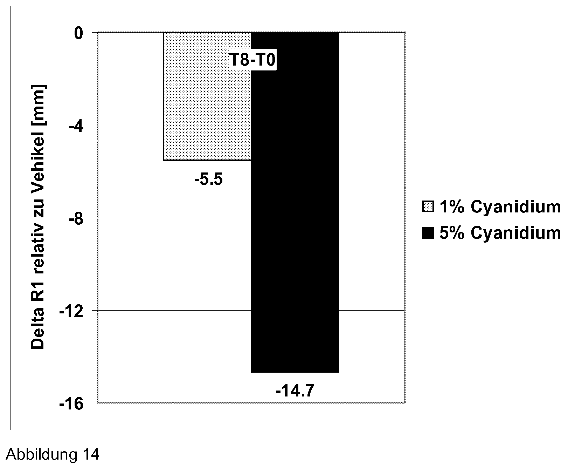

Ein ähnlicher Trend wurde für den Hautelastizitätsparameter R4 gefunden. R4 ist die Minimumamplitude des letzten der drei Dehnungszyklen der Haut und macht "Ermüdungserscheinungen" der Haut sichtbar, da die Fähigkeit zur Redeformation sich mit jeder neuen Dehnung verringert (Abbildung 14).

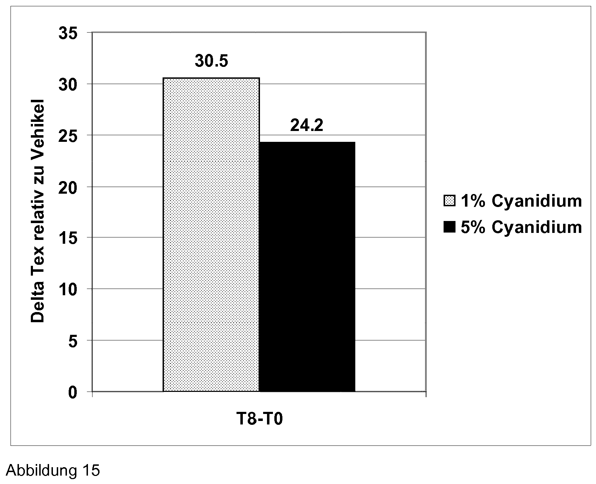

Weiterhin wurde eine Erhöhung des Haut Texturparameters Tex gefunden. Der Tex-Parameter beschreibt die Ebenmäßigkeit des Hauttons. Ein verbesserter Tex-Wert geht einher mit einem verbesserten Erscheinungsbild der Haut. Sowohl in der 1% *Cyanidium-* als auch in der 5% *Cyanidium*-Gruppe wurde ein starker Anstieg des Wertes des Tex-Parameters um 30 bzw. 24 Einheiten nach acht Wochen gefunden (Abbildung 15).

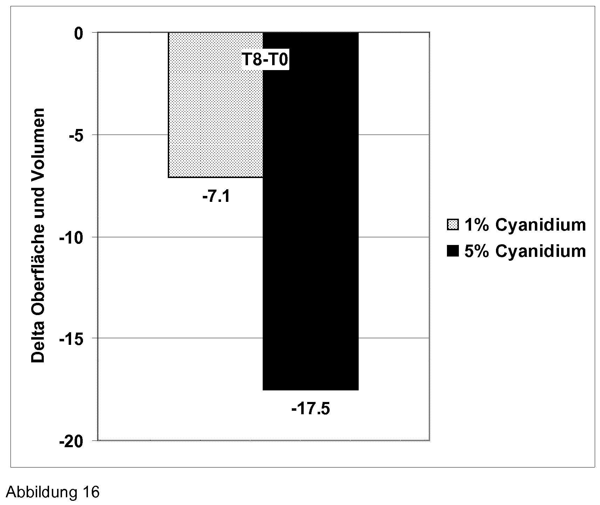

Letztendlich wurde eine Abnahme des Hautoberflächen-Parameters und des Volumen-Parameters gefunden. Der Hautoberfläche-Parameter beschreibt die Gesamtoberfläche der vermessenen Haut, während der Volumen-Parameter das Volumen repräsentiert, welches nötig ist, um die Hautfalten zu füllen. Im Falle einer Reduktion der Tiefe und Anzahl von Falten sollten der Hautoberflächen-Parameter und der Volumen-Parameter abnehmen.
Es wurde eine Abnahme des Hautoberflächen-Parameters und des Volumen-Parameters in der 1% *Cyanidium*-Gruppe um 7 Einheiten gefunden, während dieser Wert mit 15 Einheiten in der 5% *Cyanidium*-Gruppe stärker verringert war (Abbildung 16).

Die Ergebnisse der humanen *in vivo*-Studie zeigen, dass die Verwendung der erfindungsgemäßen kosmetischen Formulierung zu einer Verstärkung der Hautelastizität und Hautstraffheit, einer Verbesserung der Hautstruktur und einer Verminderung der Tiefe und Anzahl an Falten führt.

### Beispielformulierungen

**Beispielformulierung After Shave Lotion**

| Rohstoff | INCI | Massenprozent |
|---|---|---|
| ABIL® Care 85 | Bis-PEG/PPG-16/16 PEG/PPG16/16 Dimethicone; Caprylic/Capric Triglyceride | 1.5 |
| TEGOSOFT® CT | Caprylic/Capric Triglyceride | 3.0 |
| Cyclomethicone | Cyclomethicone | 3.0 |
| Tocopheryl Acetate | Tocopheryl Acetate | 0.5 |
| Frescolate ML | Menthyl Lactate, H&R | 0.5 |
| TEGO® SMO 80 V | Polysorbate 80 | 0.5 |
| Wasser | Water | ad 100 |
| *Cyanidium caldarium-*Extrakt | | 1.1 |
| Glycerin | | 2.0 |
| Ethanol | | 15.0 |
| TEGO® Carbomer 141 | Carbomer | 0.3 |
| Xanthan Gum | Xanthan Gum | 0.1 |
| Mineral Oil (30 mPas) | Mineral Oil | 1.6 |
| Natriumhydroxid (10 % in Wasser) | | q.s. |
| Konservierungsmittel, Parfüm | | q.s. |

**Beispielformulierung O/W After Shave Balm**

| Rohstoff | INCI | Massenprozent |
|---|---|---|
| AXOL® C 62 Pellets | Glyceryl Stearate Citrate | 1.50 |
| TEGO® Alkanol 1618 | Cetearyl Alcohol | 1.00 |
| TEGOSOFT® CT | Caprylic/Capric Triglyceride | 5.00 |
| TEGOSOFT® P | Isopropyl Palmitate | 2.00 |
| Cyclomethicone | Cyclomethicone | 5.50 |
| Glycerin | Glycerin | 5.00 |
| *Cyanidium caldarium*-Extrakt | | 2.0 |
| Wasser | | ad 100 |
| | | |
| TEGO® Carbomer 141 | Carbomer | 0.20 |
| TEGOSOFT® P | Isopropyl Palmitate | 0.80 |
| Natriumhydroxid (10 % in Wasser) | | q.s. |
| Konservierungsmittel, Parfüm | | q.s. |

**Beispielformulierung PEG- und Sulfat-freies konditionierendes Shampoo**

| Rohstoff | INCI | Massenprozent |
|---|---|---|
| REWOTERIC® AM C | Sodium Cocoamphoacetate | 15.0 |
| REWOPOL® SB F 12 P | Disodium Lauryl Sulfosuccinate | 3.8 |
| Wasser | Water | ad 100 |
| *Cyanidium caldarium*-Extrakt | | 0.5 |
| TEGO® Betain F 50 | Cocamidopropyl Betaine | 10.0 |
| VARISOFT® PATC | Palmitamidopropyltrimonium Chloride | 2.3 |
| REWOMID® SPA | Isostearamide MIPA | 1.0 |
| Zitronensäure | Citric Acid | q.s. |
| Konservierungsmittel, Parfüm | | q.s. |

**Beispielformulierung Sprühbare Haarmilch, PEG-frei**

| Rohstoff | INCI | Massenprozent |
|---|---|---|
| Lactic Acid, 80% | Lactic Acid | 0.40 |
| Wasser | Water | ad 100 |
| *Cyanidium caldarium*-Extrakt | | 1.50 |
| TEGO® Amid S 18 | Stearamidopropyl Dimethylamine | 1.20 |
| TEGIN® G 1100 Pellets | Glycol Distearate | 0.60 |
| TEGO® Care PS | Methyl Glucose Sesquistearate | 1.20 |
| TEGOSOFT® DEC | Diethylhexyl Carbonate | 0.30 |
| Konservierungsmittel, Parfüm | | q.s. |

**Beispielformulierung Leave-In Conditioning Foam mit Hitzeschutzwirkung**

| Rohstoff | INCI | Massenprozent |
|---|---|---|
| ABIL® T Quat 60 | Silicone Quaternium-22 | 0.5 |
| TAGAT® CH 40 | PEG-40 Hydrogenated Castor Oil | 0.5 |
| TEGO® Betain 810 | Capryl/Capramidopropyl Betaine | 2.0 |
| Wasser | Water | ad 100 |
| *Cyanidium caldarium*-Extrakt | | 1.0 |
| TEGOCEL® HPM 50 | Hydroxypropyl Methylcellulose | 0.3 |
| VARISOFT® 300 | Cetrimonium Chloride | 1.3 |
| Zitronensäure, 30% | | q.s. |
| Konservierungsmittel, Parfüm | | q.s. |

**Beispielformulierung Sun Protective Caring Body Lotion**

| Rohstoff | INCI | Massenprozent |
|---|---|---|
| TEGO® Care 165 | Glyceryl Stearate; PEG-100 Stearate | 3.5 |
| TEGIN® M Pellets | Glyceryl Stearate | 3.0 |
| TEGO® Alkanol 18 | Stearyl Alcohol | 2.0 |
| TEGOSOFT® XC | Phenoxyethyl Caprylate | 8.0 |
| Sylvaclear WF1500V | Polyamide-4, Arizona Chemical | 4.0 |
| TEGOSOFT® APM | PPG-3 Myristyl Ether | 2.0 |
| Ethylhexyl Methoxycinnamate | Ethylhexyl Methoxycinnamate | 3.0 |
| Benzophenone-3 | Benzophenone-3 | 5.0 |
| Octocrylene | Octocrylene | 7.0 |
| Menthyl Anthranilate | Menthyl Anthranilate | 4.0 |
| TEGO® Sun T 805 | Titanium Dioxide; Trimethoxycaprylylsilane | 4.0 |
| Xanthan Gum | Xanthan Gum | 0.2 |
| Glycerin | Glycerin | 2.0 |
| Wasser | Water | ad 100 |
| *Cyanidium caldarium*-Extrakt | | 1.0 |
| Allantoin | Allantoin | 0.1 |
| Tromethamin, 20 % in Wasser, Merck | Tris(hydroxymethyl)aminomethan | q.s. |
| Konservierungsmittel, Parfüm | | q.s. |

**Beispielformulierung Sun Care Soft Cream (Cold Processed) SPF 40+**

| Rohstoff | INCI | Massenprozent |
|---|---|---|
| ABIL® Care XL 80 | Bis-PEG/PPG-20/5 PEG/PPG-20/5 Dimethicone; Methoxy PEG/PPG-25/4 Dimethicone; Caprylic/Capric Triglyceride | 2.5 |
| TEGO® Sun TDEC 45 | Titanium Dioxide; Diethylhexyl Carbonate; Polyglyceryl-6 Polyhydroxystearate | 11.0 |
| Ethylhexyl Methoxycinnamate | | 8.0 |
| Octocrylene | | 7.5 |
| Butyl Methoxydibenzoylmethane | | 3.0 |
| Tocopheryl Acetate | | 0.5 |
| TEGO® Carbomer 141 | Carbomer | 0.25 |
| TEGO® Carbomer 341 ER | Acrylates / C10-30 Alkyl Acrylate Crosspolymer | 0.25 |
| Xanthan Gum | | 0.2 |
| TEGOSOFT® P | Isopropyl Palmitate | 2.0 |
| Wasser | Water | ad 100 |
| Glycerin | | 3.0 |
| *Cyanidium caldarium-*Extrakt | | 1.0 |
| Natriumhydroxid (10 % in Wasser) | | q.s. |
| Konservierungsmittel, Parfüm | | q.s. |

**Beispielformulierung After Sun Foam**

| Rohstoff | INCI | Massenprozent |
|---|---|---|
| TEGINACID® H | Glyceryl Stearate; Ceteth-20 | 3.60 |
| TEGO® Alkanol 18 | Stearyl Alcohol | 1.20 |
| TEGOSOFT® CT | Caprylic/Capric Triglyceride | 3.60 |
| TEGOSOFT® liquid | Cetearyl Ethylhexanoate | 1.80 |
| TEGOSOFT® P | Isopropyl Palmitate | 1.80 |
| TEGOSOFT® DO | Decyl Oleate | 3.00 |
| Glycerin | | 1.80 |
| Panthenol | | 0.60 |
| Allantoin | | 0.10 |
| Wasser | Water | ad 100 |
| *Cyanidium caldarium*-Extrakt | | 1.2 |
| TEGO® Betain 810 | Capryl/Capramidopropyl Betaine | 7.00 |
| Natriumhydroxid (10 % in Wasser) | | q.s. |
| Konservierungsmittel, Parfüm | | q.s. |

**Beispielformulierung O/W Cream**

| Rohstoff | INCI | Massenprozent |
|---|---|---|
| TEGO® Care PSC 3 | Polyglyceryl-3 Dicitrate/Stearate | 3.00 |
| TEGIN® M Pellets | Glyceryl Stearate | 1.20 |
| TEGO® Alkanol 18 | Stearyl Alcohol | 0.80 |
| TEGOSOFT® P | Isopropyl Palmitate | 7.00 |
| Prunus Amygdalus Dulcis Oil | | 5.00 |
| TEGOSOFT® CT | Caprylic/Capric Triglyceride | 4.50 |
| TEGOSOFT® TIS | Triisostearin | 3.50 |
| Wasser | Water | ad 100 |
| *Cyanidium caldarium-*Extrakt | | 2.0 |
| Glycerin | | 3.00 |
| Keltrol CG-SFT, CP Kelco | Xanthan Gum | 0.30 |
| | | |
| Natriumhydroxid (10 % in Wasser) | | q.s. |
| Konservierungsmittel, Parfüm | | q.s. |

**Beispielformulierung Anti-Cellulite Body Lotion**

| Rohstoff | INCI | Massenprozent |
|---|---|---|
| TEGO® Care LTP | Sorbitan Laurate; Polyglyceryl-4 | 1.50 |
| | Laurate; Dilauryl Citrate | |
| TEGOSOFT® CI | Cetearyl Isononanoate | 10.00 |
| TEGOSOFT® DEC | Diethylhexyl Carbonate | 3.50 |
| TEGOSOFT® OP | Ethylhexyl Palmitate | 1.10 |
| TEGO® Carbomer 140 | Carbomer | 0.15 |
| TEGO® Carbomer 141 | Carbomer | 0.15 |
| Xanthan Gum | | 0.10 |
| Glycerin | | 3.00 |
| Wasser | Water | ad 100 |
| *Cyanidium caldarium*-Extrakt | | 3.0 |
| Natriumhydroxid (10 % in Wasser) | | q.s. |
| Konservierungsmittel, Parfüm | | q.s. |

**Beispielformulierung Moisturizing Anti-Cellulite Cream Gel**

| Rohstoff | INCI | Massenprozent |
|---|---|---|
| TEGOSOFT® DC | Decyl Cocoate | 8.00 |
| TEGOSOFT® OP | Ethylhexyl Palmitate | 5.00 |
| TEGOSOFT® CR | Cetyl Ricinoleate | 2.00 |
| TEGO® Alkanol 1618 | Cetearyl Alcohol | 1.00 |
| Tocopheryl Acetate | | 0.50 |
| TEGOSOFT® PSE 141 G | Sucrose Stearate | 2.00 |
| TEGO® Care CG 90 | Cetearyl Glucoside | 0.50 |
| Glycerin | | 4.00 |
| Panthenol | | 0.50 |
| Wasser | Water | ad 100 |
| *Cyanidium caldarium*-Extrakt | | 5.6 |
| TEGO® Carbomer 341 ER | Acrylates / C10-30 Alkyl Acrylate Crosspolymer | 0.45 |
| Natriumhydroxid (10 % in Wasser) | | q.s. |
| Konservierungsmittel, Parfüm | | q.s. |

**Beispielformulierung New generation anti-aging moisturizer**

| Rohstoff | INCI | Massenprozent |
|---|---|---|
| TEGO® Care 450 | Polyglyceryl-3 Methylglucose Distearate | 3.00 |
| TEGIN® M Pellets | Glyceryl Stearate | 2.50 |
| TEGO® Alkanol 18 | Stearyl Alcohol | 1.50 |
| TEGOSOFT® MM | Myristyl Myristate | 1.00 |
| TEGOSOFT® DO | Decyl Oleate | 8.00 |
| TEGOSOFT® OS | Ethylhexyl Stearate | 9.00 |
| Phytosphingosine SLC | Salicyloyl Phytosphingosine | 0.10 |
| Glycerin | | 3.00 |
| Wasser | Water | ad 100 |
| *Cyanidium caldarium*-Extrakt | | 5.0 |
| TEGO® Carbomer 134 | Carbomer | 0.20 |
| TEGOSOFT® P | Isopropyl Palmitate | 0.80 |
| Natriumhydroxid (10 % in Wasser) | | q.s. |
| Konservierungsmittel, Parfüm | | q.s. |

**Beispielformulierung W/O Lotion**

| Rohstoff | INCI | Massenprozent |
|---|---|---|
| ISOLAN® GPS | Polyglyceryl-4 Diisostearate / Polyhydroxystearate / Sebacate | 3.0 |
| Paracera W 80, Paramelt B.V. | Microcrystalline Wax | 0.1 |
| Hydrogenated Castor Oil | | 0.1 |
| TEGOSOFT® DEC | Diethylhexyl Carbonate | 7.0 |
| TEGOSOFT® TIS | Triisostearin | 3.0 |
| Cyclomethicone | | 7.0 |
| Glycerin | | 3.0 |
| Magnesium Sulfate Heptahydrate | | 1.0 |
| TEGO® Cosmo C 100 | Creatine | 1.0 |
| Skinmimics® | Ceteareth-25; Glycerin; Cetyl Alcohol; Behenic Acid; Cholesterol; Ceramide NP; Ceramide NS; Ceramide EOS; Ceramide EOP; Ceramide AP; Caprooyl Phytosphingosine;Caprooyl Sphingosine | 5.0 |
| Wasser | Water | ad 100 |
| *Cyanidium caldarium*-Extrakt | | 4.5 |
| Konservierungsmittel, Parfüm | | q.s. |

**Beispielformulierung Winter Skin Moisturizer**

| Rohstoff | INCI | Massenprozent |
|---|---|---|
| ABIL® EM 90 | Cetyl PEG/PPG-10/1 Dimethicone | 2.0 |
| Hydrogenated Castor Oil | | 0.5 |
| Paracera W 80, Paramelt B.V. | Microcrystalline Wax | 0.5 |
| TEGOSOFT® CT | Caprylic/Capric Triglyceride | 10.0 |
| TEGOSOFT® HP | Isocetyl Palmitate | 10.0 |
| TEGOSOFT® DEC | Diethylhexyl Carbonate | 5.0 |
| Wasser | Water | ad 100 |
| Natriumchlorid | Sodium Chloride | 0.5 |
| *Cyanidium caldarium*-Extrakt | | 15 |
| Konservierungsmittel, Parfüm | | q.s. |

## Patentansprüche

1. Verfahren zur Herstellung von 4-Aminobuttersäure umfassend die Verfahrensschritte
A) Kultivieren von *Cyanidium caldarium*-Zellen in einem wässrigen Medium,
B) Aufschließen der *Cyanidium caldarium*-Zellen, gegebenenfalls
C) Abtrennen der festen Zellbestandteile unter Erhalt eines Extraktes und gegebenenfalls
D) weiteres Aufreinigen der 4-Aminobuttersäure.

2. Verfahren zur Herstellung eines Extraktes enthaltend 4-Aminobuttersäure umfassend die Verfahrensschritte
A) Kultivieren von *Cyanidium caldarium*-Zellen in einem wässrigen Medium,
B) Aufschließen der *Cyanidium caldarium*-Zellen, gegebenenfalls
C) Abtrennen der festen Zellbestandteile unter Erhalt eines Extraktes.

3. Verfahren zur Herstellung einer kosmetischen Formulierung enthaltend 4-Aminobuttersäure umfassend die Verfahrensschritte
A) Kultivieren von *Cyanidium caldarium*-Zellen in einem wässrigen Medium,
B) Aufschließen der *Cyanidium caldarium*-Zellen, gegebenenfalls
C) Abtrennen der festen Zellbestandteile unter Erhalt eines Extraktes und
E) Einarbeiten der aufgeschlossenen *Cyanidium caldarium*-Zellen und/oder des Extraktes in eine kosmetische Formulierung.

4. Verfahren gemäß mindestens einem der vorherigen Ansprüche, **dadurch gekennzeichnet, dass** der Extrakt in Verfahrensschritt C ein wässriger Extrakt ist.

5. Verfahren gemäß mindestens einem der vorherigen Ansprüche, **dadurch gekennzeichnet, dass** die *Cyanidium caldarium*-Zellen in Verfahrensschritt A) bei einer erhöhten Temperatur kultiviert werden.

6. Verfahren gemäß mindestens einem der vorherigen Ansprüche, **dadurch gekennzeichnet, dass** das wässrige Medium in Verfahrensschritt A) einen pH-Wert bei 25 °C von 1,5 bis 6 aufweist.

7. Verfahren gemäß mindestens einem der vorherigen Ansprüche, **dadurch gekennzeichnet, dass** der Zellaufschluss in Verfahrensschritt B) in einem wässrigen Medium erfolgt.

8. Verfahren gemäß mindestens einem der vorherigen Ansprüche, **dadurch gekennzeichnet, dass** der Zellaufschluss in Verfahrensschritt B) ein Heisswasseraufschluss kombiniert mit einem enzymatischen Zellaufschluss darstellt.

9. Verfahren gemäß mindestens einem der vorherigen Ansprüche, **dadurch gekennzeichnet, dass** das Abtrennen der festen Zellbestandteile in Verfahrensschritt C) durch Zentrifugation, Sedimentation oder Filtration erfolgt.

10. Verwendung von *Cyanidium caldarium*-Zellen oder *Cyanidium caldarium-*Zellbestandteilen zur Herstellung einer kosmetischen Formulierung.

11. Kosmetische Verwendung von *Cyanidium caldarium*-Zellen oder *Cyanidium caldarium*-Zellbestandteilen in einer kosmetischen Formulierung auf empfindlicher, trockener, juckender und/oder schuppiger Haut.

12. Kosmetische Verwendung von *Cyanidium caldarium*-Zellen oder *Cyanidium caldarium*-Zellbestandteilen in einer kosmetischen Formulierung zur Verminderung der Haut-Rauhigkeit, der Haut-Schuppigkeit, der Faltentiefe, sowie eine Verstärkung der Hautelastizität, der Haut-Straffheit und der Haut-Dicke

13. Kosmetische Verwendung von *Cyanidium caldarium*-Zellen oder *Cyanidium caldarium*-Zellbestandteilen in einer kosmetischen Formulierung zur formgebenden kosmetischen Behandlungen an Kinn, Brust, Gesäß und Bauch.

14. Kosmetische Verwendung von *Cyanidium caldarium*-Zellen oder *Cyanidium caldarium*-Zellbestandteilen in einer kosmetischen Formulierung zur Steigerung der Stabilität, Widerstandskraft, Farbintensität und Belastbarkeit von Haaren.

15. Kosmetische Verwendung von *Cyanidium caldarium*-Zellen oder *Cyanidium caldarium*-Zellbestandteilen in einer kosmetischen Formulierung zur Aufrechterhaltung der Stammzellfunktionen der Haut.

## Claims

1. Process for the production of 4-aminobutyric acid, comprising the process steps
A) culturing *Cyanidium caldarium* cells in an aqueous medium,
B) disrupting the *Cyanidium caldarium* cells, if appropriate
C) removing the solid cell components to obtain an extract and, if appropriate,
D) further purifying the 4-aminobutyric acid.

2. Process for the production of an extract comprising 4-aminobutyric acid, comprising the process steps
A) culturing *Cyanidium caldarium* cells in an aqueous medium,
B) disrupting the *Cyanidium caldarium* cells, if appropriate
C) removing the solid cell components to obtain an extract.

3. Process for the preparation of a cosmetic formulation comprising 4-aminobutyric acid, comprising the process steps
A) culturing *Cyanidium caldarium* cells in an aqueous medium,
B) disrupting the *Cyanidium caldarium* cells, if appropriate
C) removing the solid cell components to obtain an extract and
E) incorporating the disrupted *Cyanidium caldarium* cells and/or the extract in a cosmetic formulation.

4. Process according to at least one of the preceding claims, **characterized in that** the extract in process step C is an aqueous extract.

5. Process according to at least one of the preceding claims, **characterized in that** the *Cyanidium caldarium* cells in process step A) are cultured at an elevated temperature.

6. Process according to at least one of the preceding claims, **characterized in that** the aqueous medium in process step A) has a pH at 25°C of from 1.5 to 6.

7. Process according to at least one of the preceding claims, **characterized in that** the cell disruption in process step B) is carried out in an aqueous medium.

8. Process according to at least one of the preceding claims, **characterized in that** the cell disruption in process step B) is a hot-water disruption in combination with an enzymatic cell disruption.

9. Process according to at least one of the preceding claims, **characterized in that** the removal of the solid cell components in process step C) is carried out by centrifugation, sedimentation or filtration.

10. Use of *Cyanidium caldarium* cells or *Cyanidium caldarium* cell components for the preparation of a cosmetic formulation.

11. Cosmetic use of *Cyanidium caldarium* cells or *Cyanidium caldarium* cell components in a cosmetic formulation on sensitive, dry, itchy and/or flaky skin.

12. Cosmetic use of *Cyanidium caldarium* cells or *Cyanidium caldarium* cell components in a cosmetic formulation for reduced roughness of the skin, reduced scaliness of the skin, reduced wrinkle depth and an enhanced skin elasticity, skin firmness and skin thickness.

13. Cosmetic use of *Cyanidium caldarium* cells or *Cyanidium caldarium* cell components in a cosmetic formulation for shaping cosmetic treatments on the chin, the chest, the buttocks and the tummy.

14. Cosmetic use of *Cyanidium caldarium* cells or *Cyanidium caldarium* cell components in a cosmetic formulation for increasing the stability, strength, colour intensity and resistance of hair.

15. Cosmetic use of *Cyanidium caldarium* cells or *Cyanidium caldarium* cell components in a cosmetic formulation for maintaining the stem cell functions of the skin.

## Revendications

1. Procédé pour la préparation d'acide 4-aminobutyrique, comprenant les étapes de procédé
A) culture de cellules de Cyanidium caldarium dans un milieu aqueux,
B) digestion des cellules de Cyanidium caldarium, le cas échéant
C) séparation des constituants cellulaires solides avec obtention d'un extrait et le cas échéant
D) autre purification de l'acide 4-aminobutyrique.

2. Procédé pour la préparation d'un extrait contenant de l'acide 4-aminobutyrique, comprenant les étapes de procédé
A) culture de cellules de Cyanidium caldarium dans un milieu aqueux,
B) digestion des cellules de Cyanidium caldarium, le cas échéant
C) séparation des constituants cellulaires solides avec obtention d'un extrait.

3. Procédé pour la préparation d'une formulation cosmétique contenant de l'acide 4-aminobutyrique, comprenant les étapes de procédé
A) culture de cellules de Cyanidium caldarium dans un milieu aqueux,
B) digestion des cellules de Cyanidium caldarium, le cas échéant
C) séparation des constituants cellulaires solides avec obtention d'un extrait et
E) incorporation des cellules digérées de Cyanidium caldarium et/ou de l'extrait dans une formulation cosmétique.

4. Procédé selon au moins l'une quelconque des revendications précédentes, **caractérisé en ce que** l'extrait dans l'étape de procédé C est un extrait aqueux.

5. Procédé selon au moins l'une quelconque des revendications précédentes, **caractérisé en ce que** les cellules de Cyanidium caldarium dans l'étape de procédé A) sont cultivées à une température augmentée.

6. Procédé selon au moins l'une quelconque des revendications précédentes, **caractérisé en ce que** le milieu aqueux dans l'étape de procédé A) présente un pH à 25°C de 1, 5 à 6.

7. Procédé selon au moins l'une quelconque des revendications précédentes, **caractérisé en ce que** la digestion cellulaire dans l'étape de procédé B) a lieu dans un milieu aqueux.

8. Procédé selon au moins l'une quelconque des revendications précédentes, **caractérisé en ce que** la digestion cellulaire dans l'étape de procédé B) représente une digestion dans l'eau chaude combinée à une digestion cellulaire enzymatique.

9. Procédé selon au moins l'une quelconque des revendications précédentes, **caractérisé en ce que** la séparation des constituants cellulaires solides dans l'étape de procédé C) a lieu par centrifugation, sédimentation ou filtration.

10. Utilisation de cellules de Cyanidium caldarium ou de constituants cellulaires de Cyanidium caldarium pour la préparation d'une formulation cosmétique.

11. Utilisation cosmétique de cellules de Cyanidium caldarium ou de constituants cellulaires de Cyanidium caldarium dans une formulation cosmétique sur une peau sensible, sèche, prurigineuse et/ou squameuse.

12. Utilisation cosmétique de cellules de Cyanidium caldarium ou de constituants cellulaires de Cyanidium caldarium dans une formulation cosmétique pour diminuer la rugosité de la peau, le caractère squameux de la peau, la profondeur des rides ainsi que pour renforcer l'élasticité de la peau, la fermeté de la peau et l'épaisseur de la peau.

13. Utilisation cosmétique de cellules de Cyanidium caldarium ou de constituants cellulaires de Cyanidium caldarium dans une formulation cosmétique pour des traitements cosmétiques de façonnage du menton, de la poitrine, des fesses et du ventre.

14. Utilisation cosmétique de cellules de Cyanidium caldarium ou de constituants cellulaires de Cyanidium caldarium dans une formulation cosmétique pour l'augmentation de la stabilité, de la résistance, de l'intensité de couleur et de la solidité de cheveux.

15. Utilisation cosmétique de cellules de Cyanidium caldarium ou de constituants cellulaires de Cyanidium caldarium dans une formulation cosmétique pour le maintien des fonctions des cellules souches de la peau.


### SEQUENCE LISTING

<110> Evonik Industries
<120> Verfahren zur Herstellung einer kosmetischen Formulierung enthaltend 4 Aminobuttersäure
<130> 201100105
<160> 42
<170> PatentIn version 3.5
<210> 1
   <211> 18
   <212> DNA
   <213> Artificial Sequence
<220>
   <223> Primer
<400> 1
   gcctcggagg aaactttg 18
<210> 2
   <211> 18
   <212> DNA
   <213> Artificial Sequence
<220>
   <223> Primer
<400> 2
   gacccatggg acctgaag 18
<210> 3
   <211> 18
   <212> DNA
   <213> Artificial Sequence
<220>
   <223> Primer
<400> 3
   gcccagtttg gcctagtg 18
<210> 4
   <211> 18
   <212> DNA
   <213> Artificial Sequence
<220>
   <223> Primer
<400> 4
   cagcagcacc gtatttag 18
<210> 5
   <211> 19
   <212> DNA
   <213> Artificial Sequence
<220>
   <223> Primer
<400> 5
   tgggagcaaa cacatctga 19
<210> 6
   <211> 20
   <212> DNA
   <213> Artificial Sequence
<220>
   <223> Primer
<400> 6
   atcacttctc cccgaatcgt 20
<210> 7
   <211> 20
   <212> DNA
   <213> Artificial Sequence
<220>
   <223> Primer
<400> 7
   tcgcccggca gcaaactcag 20
<210> 8
   <211> 20
   <212> DNA
   <213> Artificial Sequence
<220>
   <223> Primer
<400> 8
   tcacaccgct cacaggggct 20
<210> 9
   <211> 20
   <212> DNA
   <213> Artificial Sequence
<220>
   <223> Primer
<400> 9
   caggctagag aagcagagcc 20
<210> 10
   <211> 20
   <212> DNA
   <213> Artificial Sequence
<220>
   <223> Primer
<400> 10
   tggagatttt gggagtacgg 20
<210> 11
   <211> 18
   <212> DNA
   <213> Artificial Sequence
<220>
   <223> Primer
<400> 11
   cgtggatcct gctcacct 18
<210> 12
   <211> 20
   <212> DNA
   <213> Artificial Sequence
<220>
   <223> Primer
<400> 12
   caatggagct gttgtcaagg 20
<210> 13
   <211> 20
   <212> DNA
   <213> Artificial Sequence
<220>
   <223> Primer
<400> 13
   ggttgagctg gatctgtcct 20
<210> 14
   <211> 20
   <212> DNA
   <213> Artificial Sequence
<220>
   <223> Primer
<400> 14
   agtaggataa tggccccagg 20
<210> 15
   <211> 20
   <212> DNA
   <213> Artificial Sequence
<220>
   <223> Primer
<400> 15
   accacagtcc atgccatcac 20
<210> 16
   <211> 20
   <212> DNA
   <213> Artificial Sequence
<220>
   <223> Primer
<400> 16
   tccaccaccc tgttgctgta 20
<210> 17
   <211> 20
   <212> DNA
   <213> Artificial Sequence
<220>
   <223> Primer
<400> 17
   cctggatgac tcttgggaaa 20
<210> 18
   <211> 20
   <212> DNA
   <213> Artificial Sequence
<220>
   <223> Primer
<400> 18
   tcagccagct gtttcatgtc 20
<210> 19
   <211> 20
   <212> DNA
   <213> Artificial Sequence
<220>
   <223> Primer
<400> 19
   ctctggccca ggcagtcaga 20
<210> 20
   <211> 20
   <212> DNA
   <213> Artificial Sequence
<220>
   <223> Primer
<400> 20
   ggcgtttggg aaggttggat 20
<210> 21
   <211> 21
   <212> DNA
   <213> Artificial Sequence
<220>
   <223> Primer
<400> 21
   cactccatga aggctgcatg g 21
<210> 22
   <211> 22
   <212> DNA
   <213> Artificial Sequence
<220>
   <223> Primer
<400> 22
   acccagtagt cttgctttgt gg 22
<210> 23
   <211> 20
   <212> DNA
   <213> Artificial Sequence
<220>
   <223> Primer
<400> 23
   accacagtcc atgccatcac 20
<210> 24
   <211> 20
   <212> DNA
   <213> Artificial Sequence
<220>
   <223> Primer
<400> 24
   tccaccaccc tgttgctgta 20
<210> 25
   <211> 22
   <212> DNA
   <213> Artificial Sequence
<220>
   <223> Primer
<400> 25
   ccaccagagc tattcccaaa ag 22
<210> 26
   <211> 20
   <212> DNA
   <213> Artificial Sequence
<220>
   <223> Primer
<400> 26
   gcggcgattc atcaggaaat 20
<210> 27
   <211> 20
   <212> DNA
   <213> Artificial Sequence
<220>
   <223> Primer
<400> 27
   tgccaagtgg gtggtataga 20
<210> 28
   <211> 21
   <212> DNA
   <213> Artificial Sequence
<220>
   <223> Primer
<400> 28
   tcagatgacg aagagcacag a 21
<210> 29
   <211> 20
   <212> DNA
   <213> Artificial Sequence
<220>
   <223> Primer
<400> 29
   atgtgaatgc caaagcgaag 20
<210> 30
   <211> 20
   <212> DNA
   <213> Artificial Sequence
<220>
   <223> Primer
<400> 30
   ctaccaacac gcccttcatt 20
<210> 31
   <211> 18
   <212> DNA
   <213> Artificial Sequence
<220>
   <223> Primer
<400> 31
   ctcttcggct tctcgctg 18
<210> 32
   <211> 19
   <212> DNA
   <213> Artificial Sequence
<220>
   <223> Primer
<400> 32
   cccgttctgt tggctctct 19
<210> 33
   <211> 17
   <212> DNA
   <213> Artificial Sequence
<220>
   <223> Primer
<400> 33
   gacctgccct cctggac 17
<210> 34
   <211> 20
   <212> DNA
   <213> Artificial Sequence
<220>
   <223> Primer
<400> 34
   gtaggttcgg caagtcctca 20
<210> 35
   <211> 20
   <212> DNA
   <213> Artificial Sequence
<220>
   <223> Primer
<400> 35
   cggcctgctg ctccttggtc 20
<210> 36
   <211> 20
   <212> DNA
   <213> Artificial Sequence
<220>
   <223> Primer
<400> 36
   caccgctgtg cctctggcat 20
<210> 37
   <211> 21
   <212> DNA
   <213> Artificial Sequence
<220>
   <223> Primer
<400> 37
   atcgtcccag gtggaagagt t 21
<210> 38
   <211> 22
   <212> DNA
   <213> Artificial Sequence
<220>
   <223> Primer
<400> 38
   atagtaacca ggcgtctcgt gg 22
<210> 39
   <211> 20
   <212> DNA
   <213> Artificial Sequence
<220>
   <223> Primer
<400> 39
   ggcctgctga gatcaaagac 20
<210> 40
   <211> 19
   <212> DNA
   <213> Artificial Sequence
<220>
   <223> Primer
<400> 40
   tctgcagaag gacattggc 19
<210> 41
   <211> 20
   <212> DNA
   <213> Artificial Sequence
<220>
   <223> Primer
<400> 41
   gtgctcgcgc tactctctct 20
<210> 42
   <211> 20
   <212> DNA
   <213> Artificial Sequence
<220>
   <223> Primer
<400> 42
   ttcaatgtcg gatggatgaa 20